# FASCICULE DE BREVET EUROPEEN

(11) **EP 3 232 794 B1**
(45) Date de publication et mention de la délivrance du brevet: **10.10.2018**
(21) Numéro de dépôt: 15823654.7
(22) Date de dépôt: 17.12.2015
(51) Int. Cl.: A21D 13/00, A21D 2/34, A21D 2/36

(54) **PRODUIT FRIT ALLÉGÉ EN MATIÈRE GRASSE ET PROCÉDÉ DE FABRICATION**
FETTARMES FRITTIERTES PRODUKT UND VERFAHREN ZUR HERSTELLUNG DAVON
LOW-FAT FRIED PRODUCT AND METHOD FOR PRODUCING SAME

(30) Priorité: 18.12.2014 FR 1462697
(43) Date de publication de la demande: 25.10.2017
(73) Titulaire: Corbion Biotech, Inc., South San Francisco, CA 94080 (US)
(72) Inventeur: BOURSIER, Thomas, 59370 Mons En Baroeul (FR); DELEBARRE, Marie, 62136 La Couture (FR); VERCRUYSSE, Anne-Sophie, 59800 Lille (FR)
(74) Mandataire: Cabinet Becker et Associés
(86) Numéro de dépôt international: PCT/FR2015/053564
(87) Numéro de publication internationale: WO 2016/097617

(56) Documents cités:
- EP-A2- 1 010 370
- WO-A1-98/41114
- US-A1- 2011 268 839
- US-A1- 2012 128 851
- BELL D A ET AL: "USE OF CELLULOSE ETHERS TO REDUCE FAT ABSORPTION AND INCREASE MOISTURE RETENTION IN YEAST-LEAVENED DOUGHNUTS", CEREAL FOODS WORLD, vol. 37, no. 7, 1992, pages 559-560, XP009186497, & 77TH ANNUAL MEETING OF THE AMERICAN ASSOCIATION OF CEREAL CHEMISTS, MINNEAPOLIS, MINNESOTA, USA, SEP ISSN: 0146-6283
- SHIH F F ET AL: "Development of low oil-uptake donuts", JOURNAL OF FOOD SCIENCE, vol. 66, no. 1, janvier 2001 (2001-01), pages 141-144, XP002745618, ISSN: 0022-1147
- KIM BUM-KEUN ET AL: "Reduction in fat uptake of doughnut by microparticulated wheat bran", INTERNATIONAL JOURNAL OF FOOD SCIENCES AND NUTRITION, vol. 63, no. 8, décembre 2012 (2012-12), pages 987-995, XP009186496,
- DATABASE WPI Week 201065 Thomson Scientific, London, GB; AN 2010-K23569 XP002745616, & KR 2010 0087513 A (UNIV IND&ACADEMIC COOP IN CHUNGNAM NAT) 5 août 2010 (2010-08-05)
- RABELO S F ET AL: "Development of cassava doughnuts enriched with Spirulina platensis biomass.", BRAZILIAN JOURNAL OF FOOD TECHNOLOGY, vol. 16, no. 1, mars 2013 (2013-03), pages 42-51, XP002745620,

## Description

### DOMAINE DE L'INVENTION

La présente invention concerne un produit frit. Plus précisément, l'invention se rapporte un produit frit obtenu par cuisson d'une composition comprenant de la farine, un liquide potable, de la levure et de la farine de microalgues et possédant une teneur en matière grasse réduite par rapport à la composition classique dudit produit frit.

L'invention concerne également le procédé de fabrication d'un tel produit frit.

### ARRIERE-PLAN TECHNOLOGIQUE

Le mot beignet est un diminutif du mot « beigne » qui date de la France du XIII^{e} siècle où il désignait une boule de pâte frite dans du beurre. L'enflement de la pâte lors de la cuisson a donné, au sens figuré, la signification actuelle du mot d'argot français « beigne : bosse suite à un coup ».

Les beignets appartiennent aux produits frits préparés à partir d'une pâte souple frite dans l'huile. Ils sont généralement sucrés mais peuvent également être salés. Ils peuvent être natures ou bien fourrés de confiture, chocolat, crème pâtissière, chantilly, foie gras, mousses salées, etc..

Plus généralement, on retrouve d'autres produits dans cette catégorie de produits, comme par exemple les donuts ou dougnuts, les churros, les chichis, les croustillons, les bugnes, les tempuras, etc.. Chaque région, et plus généralement chaque pays, a son ou ses appellations propres pour désigner ce type de produits frits.

Les beignets ou donuts sont très populaires en Amérique du Nord. La version la plus courante est de forme annulaire, à texture relativement dense, souvent recouverte d'un glaçage, qui fut popularisée dans les années 1950 par certaines chaînes de restauration rapide. Le donut a été popularisé hors de l'Amérique du Nord par la série animée « Les Simpson » comme étant le péché mignon d'Homer Simpson.

Le procédé de préparation des beignets et homologues consiste à réaliser une pâte souple, à la mettre en forme pour ensuite la plonger dans un bain d'huile. Une fois la cuisson réalisée, les beignets peuvent être saupoudrés de sucre, glacés, glacés au sucre fondu.

Par essence, les beignets sont souvent riches en glucides simples et en matières grasses, notamment saturées, du fait de l'emploi de matières grasses connues sous l'appellation « shortening » surtout au niveau industriel. Or, les recommandations de santé publiques encouragent fortement à limiter la consommation de sucre, d'aliments riches en sucre et/ou en matières grasses de type saturées.

De plus, leur procédé de préparation passant par une étape de friture renforce la charge calorique et la teneur en matière grasse de ce type de produits, pourtant très appréciés par les consommateurs, dont les adolescents et jeunes adultes. En effet, les procédés de friture apportent des matières grasses de type trans, mauvais pour le coeur.

Pourtant, les matières grasses d'origine animale et/ou végétale jouent un rôle important dans les produits destinés à la boulangerie, pâtisserie et viennoiserie. Non seulement elles révèlent et véhiculent l'arôme des produits finis, mais elles déterminent le résultat d'une série de caractéristiques techniques telles que par exemple, le moelleux, le croustillant ou le bon goût riche d'un gâteau au beurre. On les sait caloriques et selon leur origine pas forcément très bonnes pour la santé, et pourtant, impossible de s'en passer tant leur double rôle technique et gustatif est important, voire primordial sur le résultat final du produit.

De plus, il est tout de même nécessaire de consommer une certaine quantité de matières grasses quotidiennement pour assurer le bon fonctionnement de notre organisme. Par exemple, les huiles et les lipides apportent des calories et des acides gras essentiels qui aident l'organisme à absorber les vitamines liposolubles comme les vitamines A, D, E et K. Le type de lipide consommé est aussi important pour la santé que la quantité consommée.

C'est pourquoi il est fortement recommandé de choisir des lipides insaturés connus pour être de bons lipides. Consommer trop de lipides du mauvais type, comme les lipides saturés (shortening) et les lipides trans (produits frits), peut faire élever les taux de cholestérol LDL (Low Density Lipoprotein ou « mauvais » cholestérol) et réduire ceux du cholestérol HDL (High Density Lipoprotein ou « bon » cholestérol). Ce déséquilibre peut faire augmenter les risques d'hypertension artérielle, de rétrécissement des artères (athérosclérose), de crise cardiaque et d'AVC (Accident Vasculaire Cérébral).

Parmi les lipides insaturés, on distingue les lipides monoinsaturés et les polyinsaturés. Il a été démontré que les matières grasses monoinsaturées améliorent les taux de cholestérol sanguins. On les retrouve dans l'huile d'olive, de canola et d'arachide, dans la margarine non hydrogénée, dans les avocats et dans certains fruits à coques comme les noix, les amandes, les pistaches, les noix de cajou, les pacanes et les noisettes. Les matières grasses polyinsaturées aident l'organisme à se débarrasser du cholestérol récemment produit. Parmi celles-ci, on retrouve les oméga-3, qui peuvent prévenir les caillots sanguins, réduire le risque de subir un AVC et aussi réduire les triglycérides, un type de matières grasses dans le sang lié aux maladies du coeur. Les meilleures sources d'oméga-3 sont les poissons d'eau froide, de même que les huiles de canola et de soja, les oeufs riches en oméga-3, les graines de lin, les noix de Grenoble, les pacanes et les pignons. On retrouve aussi dans cette catégorie de matières grasses, les oméga-6 qui aident à réduire le cholestérol LDL, mais dont une trop grande consommation peut également réduire le cholestérol HDL. Il faut donc les consommer avec modération. On les retrouve dans les huiles de carthame, de tournesol et de maïs, les margarines non hydrogénées, les noix tels que les amandes, les pacanes, les noix du Brésil et les graines de tournesol. De nombreux plats préparés en contiennent également.

En parallèle, on trouve les lipides saturés que l'on retrouve le plus fréquemment dans les viandes grasses, les produits laitiers entiers, le beurre, le saindoux, les shortening, l'huile de noix de coco et l'huile de palme. Ces matières grasses peuvent augmenter le « mauvais » cholestérol LDL. Tout comme les matières grasses saturées, les lipides trans font augmenter le cholestérol LDL. Les lipides trans se retrouvent dans les margarines partiellement hydrogénées, les aliments frits des comptoirs de restauration rapide (frites, beignets, donuts) et dans certains craquelins, biscuits et produits de pâtisserie commerciale.

De ce qui précède, nous pouvons retenir que les matières grasses rendent les aliments plus savoureux et sont indispensables à notre santé. Mais, que consommées en excès, elles peuvent avoir des effets négatifs, notamment sur le système cardiovasculaire.

Depuis plusieurs années, les spécialistes dénoncent les gras trans comme étant encore plus dangereux pour la santé que les graisses saturées. Selon des études, supprimer tous les acides gras trans de l'alimentation entraînerait une baisse de 20% des décès causés par les maladies cardiovasculaires. Malheureusement, les gras trans sont souvent présents dans les gâteaux, les biscuits, les croustilles, les beignes, les margarines, les poissons et autres produits frits préparés.

Il est donc intéressant de chercher à limiter l'apport des lipides dits néfastes, saturés et/ou de type trans dans les produits de types beignets, donuts et homologues.

Ainsi, dans l'art antérieur, des solutions ont été décrites pour éliminer l'étape de friture sur les donuts. Le problème est que le résultat obtenu n'est pas à la hauteur. La texture des « donuts » cuits au four n'est plus du tout identique à celle recherchée. Lesdits donuts ressemblent davantage à des petits gâteaux à texture briochée.

Ainsi, les solutions connues de l'art antérieur conduisent très souvent à des produits présentant une qualité finale moindre, en termes de texture et de goût notamment.

Il existe donc un réel besoin pour fabriquer des beignets, donuts et homologues possédant une charge calorique réduite et étant par conséquent meilleurs pour la santé. Les solutions proposées doivent résulter dans des produits présentant les mêmes qualités organoleptiques que les produits dits traditionnels. Par ailleurs, les solutions proposées doivent pouvoir être mise en oeuvre par les hommes du métier sans changement drastique des recettes et de préférence à grande échelle, sur des productions en ligne.

Des beignets, donuts et homologues possédant une charge calorique réduite sont décrits par exemple dans Bell et al., Cereal Foods World, 1992, vol. 37, pages 559-560 ; Shih et al., Journal of Food Science, 2001, vol. 66, pages 141-144 ; Kim et al., International Journal of Food Technology, 2012, vol. 63, pages 987-995 ; ainsi que dans les demandes de brevets US2011/268839, WO98/41114 et EP1010370.

### RESUME DE L'INVENTION

Forte de ce constat et après de nombreux travaux de recherche, la société Demanderesse a eu le mérite de répondre à toutes les exigences requises et a trouvé qu'un tel but pouvait être atteint dès lors que l'on utilise une farine de microalgues en tant qu'ingrédient dans la formulation d'une composition pour produit frit, farine de microalgues apte à se substituer partiellement ou totalement aux matières grasses d'origine végétale et/ou animale, et plus particulièrement aux matières grasses de type saturées généralement utilisées dans la production industrielle desdits produits frits.

Il est donc du mérite de la Demanderesse d'avoir découvert qu'une farine de microalgues pouvait, de façon surprenante et inattendue par rapport aux prérequis de l'art antérieur, remplacer partiellement ou totalement les matières grasses d'origine animale et/ou végétale et plus particulièrement les matières grasses de type saturées généralement utilisées dans la production industrielle desdits produits frits, tout en conservant les qualités organoleptiques, en particulier gustatives, olfactives, visuelles et tactiles, au moins équivalentes voire supérieures, à celles des produits frits traditionnels contenant ces ingrédients.

Par conséquent, la présente invention concerne un produit frit obtenu à partir d'une composition pour produit frit comprenant de la farine, de la levure, de la farine de microalgue et un liquide potable. Selon l'invention, la composition pour produit frit, ou composition initiale, est susceptible également de contenir une matière sucrante, et de préférence du saccharose, de l'allulose et/ou leur mélanges quelconques. Selon ce mode de réalisation, ladite composition comprend jusqu'à 20%, de préférence moins de 10% d'une matière sucrante, de préférence choisie parmi le saccharose, l'allulose et/ou leurs mélanges quelconques, ces pourcentages étant exprimés par rapport à la masse totale des ingrédients mis en oeuvre dans la composition.

Toujours selon l'invention, ladite composition peut comprendre jusqu'à 80%, notamment entre 10% et 80%, préférentiellement entre 20% et 70%, de manière préférée entre 30% et 65%, et plus préférentiellement encore entre 35% et 55% de farine, ces pourcentages étant exprimés par rapport à la masse totale des ingrédients mis en oeuvre dans la composition.

Dans un mode de réalisation secondaire, la composition ne contient pas de gluten.

Selon un autre mode de réalisation de l'invention, ladite composition pour produit frit peut comprendre jusqu'à 30%, de préférence moins de 20 %, plus préférentiellement moins de 1 0%, et encore plus préférentiellement moins de 5% d'oeufs ou ovoproduits, ces pourcentages étant exprimés par rapport à la masse totale des ingrédients mis en oeuvre dans la composition.

Elle est également susceptible de contenir jusqu'à 30%, de préférence moins de 20 %, plus préférentiellement moins de 10%, et encore plus préférentiellement moins de 5% de dérivés du lait, ces pourcentages étant exprimés par rapport à la masse totale des ingrédients mis en oeuvre dans la composition.

Grâce à l'utilisation d'une farine de microalgues, la composition est également caractérisée en ce que les matières grasses d'origine végétale et/ou animale d'origine ont été remplacées partiellement ou totalement par de la farine de microalgues, et de préférence la substitution est totale.

Ladite composition se caractérise également par une teneur en matière grasse qui est réduite de 30%, de préférence de 40% et encore plus préférentiellement de 55% en comparaison de la teneur en matière grasse contenue dans la composition pour produit frit usuelle, c'est-à-dire exempte de farine de microalgues, ces pourcentages étant exprimés par rapport à la masse totale des ingrédients mis en oeuvre dans la composition.

Dans la composition selon l'invention, la matière grasse habituellement utilisée d'origine végétale et/ou animale peut être remplacée par un mélange de farine de microalgues et d'un liquide potable, pouvant être préférentiellement de l'eau, ladite eau pouvant être de l'eau de source, de l'eau minérale, gazéifiée naturellement ou par adjonction de gaz carbonique ou non gazéifiée. Ainsi, 100 parties de la matière grasse habituellement utilisée sont avantageusement remplacées par 5 à 50 parties de farine de microalgues et 50 à 95 parties d'un liquide potable, et notamment 10 parties de farine de microalgues et 90 parties d'un liquide potable, 15 parties de farine de microalgues et 85 parties d'un liquide potable, 25 parties de farine de microalgues et 75 parties d'un liquide potable, 35 parties de farine de microalgues et 65 parties d'un liquide potable.

Dans un autre mode de réalisation de l'invention, la composition pour produit frit est caractérisée en qu'elle comprend de 0,5% à 20% de farine de microalgues, de préférence de 1% à 15%, plus préférentiellement de 1% à 10%, et encore plus préférentiellement de 1% à 5%, ces pourcentages étant exprimés par rapport à la masse totale des ingrédients mis en oeuvre dans la composition.

Selon un mode de réalisation de l'invention, la composition pour produit frit peut comprendre entre 5% et 75% de liquide potable, préférentiellement entre 10% et 70%, plus préférentiellement entre 20% et 50%, ces pourcentages étant exprimés par rapport à la masse totale des ingrédients mis en oeuvre dans la composition. Le liquide potable s'entend de l'ensemble des liquides utilisés dans la composition, y compris le cas échéant le lait et les dérivés de lait liquides et les oeufs et ovoproduits liquides.

Toujours selon un mode de réalisation de l'invention, la composition est également caractérisée en ce que la somme des composants consistant en la farine de microalgues et le liquide potable représente au moins 25%, et de préférence au moins 30% en masse de la totalité des ingrédients utilisés dans la composition pour produit frit. Notamment, la somme des composants consistant en la farine de microalgues et le liquide potable peut représenter entre 5% et 75%, préférentiellement entre 10 et 60%, plus préférentiellement entre 20% et 50% en masse de la totalité des ingrédients utilisés dans la composition pour produit frit.

Selon un mode de réalisation de l'invention, la farine de microalgues contenue dans ladite composition est une farine de microalgues du genre *Chlorella,* et plus particulièrement de l'espèce *Chlorella protothecoides.*

Dans un premier mode de réalisation, la farine de microalgues est sous la forme de cellules non lysées.

Dans un second mode de réalisation, la farine de microalgues est sous la forme de cellules partiellement lysées et contient de 25% à 75% de cellules lysée.

Dans un dernier mode de réalisation, la farine de microalgues est sous la forme de cellules fortement lysées et contient 85% ou plus de cellules lysée, de préférence 90% ou plus. Ladite farine de microalgues utilisée dans la composition pour produit frit selon l'invention contient préférentiellement au moins 12%, au moins 25%, au moins 40%, au moins 50%, ou au moins 75% en poids sec de lipides.

Dans un mode de réalisation particulier de l'invention, la composition pour produit frit comprend entre 30 et 80% de farine, de la levure, entre 0.5% et 20% d'oeufs ou ovoproduits, et entre 0.5% et 20% de farine de microalgues, les pourcentages étant exprimés en masse de la totalité des ingrédients utilisés dans la composition pour produit frit. Elle peut en outre comporter entre 0.5% et 20% de matière sucrante et/ou entre 0.5% et 20% de dérivés du lait, les pourcentages étant exprimés en masse de la totalité des ingrédients utilisés dans la composition pour produit frit.

L'invention concerne également l'utilisation d'une composition telle que décrite ci-dessus pour la réalisation de produits frits.

Un autre aspect de l'invention concerne également un procédé de préparation d'un produit frit comprenant une composition telle que décrite précédemment, caractérisé en ce qu'il comprend les étapes suivantes:
- une étape de mélange de la farine, des oeufs ou ovoproduits, de la levure et de la farine de microalgues à un liquide potable, préférentiellement de l'eau ;
- une étape de fermentation de ce mélange,
- une étape de mise en forme dudit mélange en boules de pâte,
- une étape de cuisson desdites boules de pâtes dans un bain d'huile chaude entre 150°C et 200°C pendant une durée allant de 30 secondes à 3 minutes pour chaque face.

Ledit procédé selon l'invention permet d'obtenir des produits frits possédant une teneur en matières grasses totales réduite d'au moins 30%, de préférence, 40% et encore plus préférentiellement de 45% par rapport au produit frit obtenu selon une composition classique.

Dans le contexte de l'invention, une composition pour produit frit classique s'entend d'une composition exempte de farine de microalgues, qui est classiquement composée de farine, levure et d'un liquide potable.

Un troisième aspect de la présente invention concerne également un produit frit susceptible d'être obtenu en mettant en oeuvre ladite composition selon l'invention et/ou par le procédé de préparation toujours selon l'invention, caractérisé en ce qu'il comprend de la farine de microalgues et dont la teneur en matière grasse est réduite d'au moins 30%, de préférence, 40% et encore plus préférentiellement de 45% par rapport au produit frit obtenu selon une composition classique.

Plus généralement, l'invention a trait à un produit frit comprenant de la farine, de la levure, de la farine de microalgue et un liquide potable. Selon l'invention, le produit frit obtenu peut éventuellement être sucré et contenir de préférence du saccharose, de l'allulose et/ou leurs mélanges quelconques.

Toujours selon l'invention, le produit frit peut également être saupoudré de sucre, glacé, glacés au sucre fondu, recouvert d'un nappage sucré et aromatisé, et/ou fourré d'une crème pâtissière, de chantilly salée ou sucrée et/ou aromatisée, d'une ganache, d'un fourrage gras.

L'invention concerne également l'utilisation d'une farine de microalgues comme matière grasse de substitution, ou « fat replacer », dans la préparation d'une composition pour produit frit selon l'invention.

L'invention a également trait à l'utilisation de farine de microalgues pour diminuer la teneur en matière grasse dans un produit frit. Avantageusement, l'utilisation d'une telle farine de microalgues permet de diminuer la teneur en matière grasse d'au moins 30%, de préférence, 40% et encore plus préférentiellement de 45% par rapport au produit frit dépourvus de farine de microalgues.

L'invention propose également un procédé de réduction de la teneur en matière grasse dans un produit frit, selon lequel on remplace au moins partiellement la farine de la composition pour produit frit par de la farine de microalgues.

### DESCRIPTION DETAILLEE DES MODES DE REALISATION

Ainsi, la présente invention concerne une composition pour produit frit comprenant de la farine, de la levure, de la farine de microalgue et un liquide potable.

Dans la présente invention, la dénomination « produit frit » utilisée doit être comprise dans son interprétation la plus large et comme désignant un produit constitué d'un mélange à minima de farine, de levure et de matière grasse, et dont le mélange est ensuite mis à frire par petite quantité de pâte préférentiellement dans un bain d'huile chaude entre 150°C et 250°C pendant une durée allant de 2 secondes à 3 minutes pour chaque face. Ledit produit frit peut également être frit après avoir été vaporisé d'huile, déposé dans un plat et soumis à des radiations infrarouge ou encore être frit après avoir été vaporisé d'huile, déposé dans un plat et soumis à une cuisson dans un four.

Dans la présente invention, la dénomination « huile » utilisée doit être comprise dans son interprétation la plus large et comme désignant des triglycérides, diglycérides, monoglycérides, phospholipides et/ou leurs mélanges quelconques. L'huile peut être solide ou liquide à température ambiante.

Ainsi au sens de la présente invention, la dénomination « produit frit » désigne particulièrement les produits de type beignets, donuts, churros, chichis, croustillons, bugnes, tempuras, etc..

Dans un mode de réalisation préféré de l'invention, la composition pour produit frit est caractérisée en ce qu'elle comprend également une matière sucrante, et de préférence du saccharose, de l'allulose et/ou leurs mélanges quelconques.

Selon ce mode de réalisation, la composition selon l'invention est caractérisée en ce qu'elle comprend jusqu'à 20%, de préférence moins de 15%, plus préférentiellement moins de 10%, et de manière encore préférée moins de 5% d'une matière sucrante, de préférence choisie parmi le saccharose, l'allulose et/ou leurs mélanges quelconques, ces pourcentages étant exprimés par rapport à la masse totale des ingrédients mis en oeuvre dans la composition. Par exemple, la composition comprend entre 0.5% et 20%, préférentiellement entre 1% et 15%, plus préférentiellement entre 1% et 10%, et de manière encore préférée entre 1% et 5% d'une matière sucrante.

Le saccharose, ou sucrose, est un disaccharide constitué d'une molécule de glucose unie à une molécule de fructose par une liaison glycosidique. C'est le sucre de table extrait de la canne à sucre ou de la betterave à sucre.

L'allulose ou psicose est un cétohexose constitué d'une chaîne de cinq éléments carbone ainsi que d'une fonction cétone, possédant un fort pouvoir sucrant.

Dans un mode de réalisation secondaire, la composition pour produit frit selon l'invention peut également contenir une autre matière sucrante, choisie par exemple parmi le glucose (ou dextrose), le sirop de glucose, le sucre inverti, le fructose ou lévulose, le lactose, le maltose, le miel, le sirop d'érable, les édulcorants intenses, les polyols tels le sorbitol ou le maltitol, l'isomalt, le stévia et/ou leurs mélanges quelconques.

Selon l'invention, la composition pour produit frit est caractérisée en ce qu'elle comprend jusqu'à 80%, et notamment entre 10% et 80%, préférentiellement entre 20% et 70%, plus préférentiellement entre 30% et 65%, et plus préférentiellement encore entre 35% et 55% de farine, ces pourcentages étant exprimés par rapport à la masse totale des ingrédients mis en oeuvre dans la composition.

Dans un mode de réalisation de la présente invention, la farine utilisée dans lesdites compositions pour produit frit est sous la forme d'une poudre obtenue en broyant et en moulant des céréales. Elle désigne en général les farines de blé, c'est-à-dire les farines classiques de meunerie, de la farine blanche à la farine complète.

Dans un mode secondaire de réalisation de l'invention, la farine utilisée est une farine qui ne contient pas de gluten, et qui peut être notamment choisie parmi les farines de riz, de châtaigne, de lupin, de pois chiche, de sarrasin, de maïs, de quinoa, de coco, de souchet, de pépin de raisin, de millet, de chanvre et leurs mélanges quelconques.

Dans un autre mode de réalisation, la farine utilisée peut être obtenue à partir de matières premières contenant généralement du gluten mais ayant été rendues « exemptes de gluten » par des traitements spéciaux bien connus de l'homme du métier. Par exemple, le gluten peut être extrait des farines en contenant naturellement par lavage de l'amidon. La pâte obtenue est rincée et malaxée jusqu'à ce que l'eau de rinçage devienne claire et soit exempte d'amidon. Ainsi, la farine peut également être de toutes origines botaniques contenant du gluten, à condition de subir un procédé particulier d'élimination du gluten. Ainsi, les farines dérivées de blé (ou froment ou épeautre), d'orge, de seigle ou de triticale (blé + seigle) sont également utilisables, à condition qu'elles soient bien dénuées de gluten (gluten free) après les procédés d'extraction mis en oeuvre.

Ainsi, dans un mode de réalisation secondaire l'invention, la composition pour produit frit ne contient pas de gluten.

Le gluten est un mélange de protéines combiné avec de l'amidon dans l'endosperme de la plupart des céréales. Il constitue environ 80% des protéines contenues dans le blé. Le gluten se divise en deux groupes : les prolamines (gliadines dans le blé), responsables de la maladie coeliaque et de l'intolérance très pernicieuses et les gluténines.

Dans un autre mode de réalisation de l'invention, la composition pour produit frit contient des oeufs ou ovoproduits.

Selon l'invention, la composition pour produit frit peut alors comprendre jusqu'à 30%, de préférence moins de 15%, plus préférentiellement moins de 10%, et encore plus préférentiellement moins de 5% d'oeufs ou ovoproduits, ces pourcentages étant exprimés par rapport à la masse totale des ingrédients mis en oeuvre dans la composition. Par exemple, la composition comprend entre 0.5% et 30%, préférentiellement entre 1% et 15%, plus préférentiellement entre 1% et 10%, et de manière encore préférée entre 1% et 5% d'oeufs ou ovoproduits.

Dans la présente invention, le terme « oeufs ou ovoproduits » doit être compris dans son interprétation la plus large et comme désignant par exemple, et de manière non limitative, les oeufs entiers incluant ceux possédant une coquille blanche ou brune et de toute origine animale, tout comme les substituts d'oeufs incluant les dérivés d'oeufs comme par exemple et sans limitation les blancs d'oeufs (albumine), les jaunes d'oeuf, et pouvant être sous différentes formes, comme concentrée, congelée, poudreuse, liquide, atomisée, etc.

Dans un mode de réalisation de l'invention, les oeufs ou ovoproduits sont sous forme atomisée.

En panification et boulangerie, les oeufs sont utilisés pour améliorer la saveur et la couleur des produits. Ils assouplissent également la pâte, grâce notamment à la lécithine qu'ils contiennent. Ils ont également un rôle hydratant au niveau de la farine et créent l'humidité nécessaire à la fermentation de la pâte. Enfin, ils permettent d'augmenter le volume des produits finis.

Selon l'invention, la composition pour produit frit peut également contenir des dérivés du lait.

Selon l'invention, la composition pour produit frit peut également comprendre jusqu'à 30%, de préférence moins de 10%, et encore plus préférentiellement moins de 5% de dérivés du lait, ces pourcentages étant exprimés par rapport à la masse totale des ingrédients mis en oeuvre dans la composition. Par exemple, la composition comprend entre 0.5% et 30%, préférentiellement entre 1% et 15%, plus préférentiellement entre 1% et 10%, et de manière encore préférée entre 1% et 5% de dérivés du lait.

Dans la présente invention, le terme « lait ou dérivés du lait » doit être compris dans son interprétation la plus large et comme désignant par exemple, et de manière non limitative tout produit obtenu à la suite d'un traitement quelconque du lait, qui peut contenir des additifs alimentaires et autres ingrédients fonctionnellement nécessaires au traitement (définition du CODEX Alimentarius). Ce sont par exemple des ingrédients laitiers fondamentaux comme des poudres de lait écrémé ou entier, des caséines et caséinates, des produits de lactosérum comme les lactosérums doux ou acides, les protéines sériques, les perméats.

Au niveau légal, il n'existe qu'une définition claire datant de 1909 définissant le lait d'origine animale : « Le lait est le produit intégral de la traite totale et ininterrompue d'une femelle laitière bien portante, bien nourrie et non surmenée. Il doit être recueilli proprement et ne pas contenir de colostrum. » La dénomination « lait » sans indication de l'espèce animale de provenance est, au niveau de la législation française, réservée au lait de vache. Tout lait provenant d'une femelle laitière autre que la vache doit être désigné par la dénomination « lait » suivie de l'indication de l'espèce animale dont il provient, par exemple « lait de chèvre », « lait de brebis », « lait d'ânesse », « lait de bufflonne », etc. Toutefois, au sens de la présente invention, le lait et les produits laitiers peuvent avoir pour origine n'importe quelle espèce animale.

En panification et boulangerie, le lait a une action bénéfique sur la pâte et un effet positif sur plusieurs phases de fabrication des produits. Il améliore la structure et l'hydratation des pâtes, favorise et régularise la fermentation, améliore la cuisson de la pâte et la saveur ainsi que la coloration des produits à la cuisson. Il apporte également du moelleux à la mie, et augmente la durée de conservation des produits finis. Le lait est le second élément liquide utilisé par le boulanger.

Le lait est également connu comme étant un agent gustatif. Il sucre légèrement les pâtes et adoucit les goûts et permet également de fixer les arômes. C'est un très bon agent de texture. Il donne de la souplesse aux pâtes.

Dans un mode de réalisation de l'invention, le lait ou dérives du lait sont sous forme atomisée.

Traditionnellement, les compositions pour produits frits de type beignets ou donuts contiennent de la matière grasse de type « shortening ». Ceci se vérifie notamment pour toutes les compositions pour produits frits industriels.

Le shortening est une graisse végétale partiellement liquide et partiellement solide à une température de 10°C, pouvant par exemple être choisie parmi l'huile de soja, l'huile de fleur de tournesol, l'huile de graine de coton, l'huile de palme, l'huile de coco, l'huile de colza, l'huile de copra et leurs dérivés et/ou leurs mélanges quelconques. Le shortening appartient donc aux matières grasses qualifiées de végétales.

Les matières grasses de type shortening n'ont pas bonne presse car elles appartiennent à la catégorie des lipides saturés qui contribuent à augmenter le mauvais cholestérol de type LDL, augmentant ainsi les risques de maladies cardiovasculaires.

Occasionnellement, les compositions pour produits frits peuvent également contenir de la matière grasse sous d'autres formes, telles que du beurre, de la margarine ou de l'huile.

Dans la présente invention, le terme « matière grasse» doit être compris dans son interprétation la plus large et comme désignant par exemple, et de manière non limitative, tout produit choisi parmi les beurres, les margarines, les huiles ou la farine de microalgues et/ou leurs mélanges quelconques.

Dans la présente invention, le terme « matière grasse d'origine végétale et/ou animale » doit être compris dans son interprétation la plus large et comme désignant par exemple, et de manière non limitative, tout produit choisi parmi les beurres, les margarines ou les huiles et/ou leurs mélanges quelconques.

Un avantage de la présente invention est la capacité de la farine de microalgues à remplacer totalement ou partiellement les matières grasses d'origine végétale et/ou animale habituellement utilisées, tout en conservant les qualités organoleptiques du produit frit, voire en les améliorant. En outre, cette substitution peut être faite sans changement, tout du moins substantiel, des recettes de préparation des compositions pour produits frits.

Selon un mode de réalisation de l'invention, la composition pour produit frit est caractérisée en ce que les matières grasses d'origine végétale et/ou animale ont été remplacées partiellement ou totalement par de la farine de microalgues.

Selon un mode de réalisation particulièrement préféré, la composition pour produit frit est caractérisée en ce que les matières grasses d'origine végétale et/ou animale ont été remplacées totalement par de la farine de microalgues. La substitution des matières grasses d'origine végétale et/ou animale est donc totale.

Par « totalement » est entendu que la composition pour produit frit selon la présente invention ne comprend pas les ingrédients remplacés, de préférence même à l'état de trace. Par « partiellement » est entendu que, en comparaison de la recette utilisée, la teneur en l'ingrédient remplacé est diminuée d'au moins 10, 20, 30, 40, 50, 60, 70, 80 ou 90 % en poids, par exemple d'environ 10, 20, 30, 40, 50, 60, 70, 80 ou 90 % en poids.

Par « environ » est entendu la valeur plus ou moins 10 % de celle-ci, de préférence plus ou moins 5 % de celle-ci. Par exemple, « environ 100 » signifie entre 90 et 110, de préférence entre 95 et 105.

En conséquence de la substitution, les compositions pour produit frit selon la présente invention ont une charge calorique inférieure à celle des produits frits conventionnels et sont donc appropriées pour la consommation par des personnes soucieuses de leur ligne, de leur forme et de leur santé.

Selon l'invention, la composition pour produit frit possède avantageusement une teneur en matière grasse réduite de 30%, de préférence de 40% et encore plus préférentiellement de 55% en comparaison de la teneur en matière grasse contenue dans la composition pour produit frit usuelle, exempte de farine de microalgues, ces pourcentages étant exprimés par rapport à la masse totale des ingrédients mis en oeuvre dans la composition. Ainsi, la teneur totale en matière grasse dans la composition selon l'invention est avantageusement comprise entre 0,5% et 25%, préférentiellement entre 1% et 15%, et de manière encore préférée entre 1,5% et 10%, ces pourcentages étant exprimés par rapport à la masse totale des ingrédients mis en oeuvre dans la composition.

Selon l'invention, la substitution totale ou partielle, mais de préférence totale de la matière grasse d'origine par de la farine de microalgues permet ainsi de réduire la teneur en matière grasse de ladite composition, et ainsi d'améliorer sa valeur calorique.

En effet, la substitution n'est pas réalisée en poids pour poids, mais en mélange avec un liquide potable. Ainsi une substitution de 100 parties de matière grasse habituellement utilisée d'origine végétale et/ou animale est remplacée par un mélange comprenant de 5 à 50 parties de farine de microalgues et de 50 à 95 parties d'un liquide potable, et notamment 10 parties de farine de microalgues et 90 parties d'un liquide potable, 15 parties de farine de microalgues et 85 parties d'un liquide potable, 25 parties de farine de microalgues et 75 parties d'un liquide potable, ou 35 parties de farine de microalgues et 65 parties d'un liquide potable. Dans un mode de réalisation particulier, 100 parties de matière grasse habituellement utilisée sont remplacées par un mélange de 25 parties de farine de microalgues et 75 parties d'un liquide potable. Du coup, la charge calorique s'en trouve forcément réduite. Avantageusement, le mélange de substitution selon l'invention comprend entre 0,5% et 25 %, préférentiellement entre 1% et 15%, en poids de farine de microalgues et entre 20% et 70%, préférentiellement entre 25% et 60%, et plus préférentiellement entre 30% et 50% en poids de liquide potable.

Il est donc tout à fait surprenant et inattendue que 100 parties de matière grasse habituellement utilisée d'origine végétale et/ou animale puissent être remplacées par un mélange de farine de microalgues et d'un liquide potable sans impacter les qualités organoleptiques finales dudit produit frit. Par ailleurs, cette substitution peut être mise en oeuvre par les hommes du métier sans changement drastique des recettes et de préférence à grande échelle, sur des productions en ligne. Ce qui fait tout l'intérêt de l'invention ici décrite.

Au sens de l'invention, le terme « liquide potable » doit être compris dans son interprétation la plus large et comme désignant par exemple, et de manière non limitative, l'eau, les jus de fruits, les nectars de fruits, les jus de légumes, les nectars de légumes, les sodas.

Selon un mode préféré de l'invention, le liquide potable est de l'eau, ladite eau pouvant être de l'eau de source, de l'eau minérale, gazéifiée naturellement ou par adjonction de gaz carbonique ou non gazéifiée.

Selon un autre mode de réalisation préféré de l'invention, le liquide potable peut également être constitué en tout ou partie par les oeufs ou ovoproduits et/ou par le lait ou dérivés du lait, étant entendu que dans ce cas ils sont sous forme liquide.

Ainsi le liquide potable peut être soit de l'eau ; soit un mélange d'eau et d'oeufs ou ovoproduits sous forme liquide ; soit un mélange d'eau et de lait ou dérivés de lait sous forme liquide ; soit un mélange d'eau, d'oeufs ou ovoproduits sous forme liquide et de lait ou dérivés de lait sous forme liquide ou soit un mélange d'oeufs ou ovoproduits sous forme liquide et de lait ou dérivés du lait sous forme liquide.

Selon un mode de réalisation de l'invention, la composition pour produit frit peut comprendre entre 5% et 75% de liquide potable, préférentiellement entre 10% et 70%, plus préférentiellement entre 20% et 50%, ces pourcentages étant exprimés par rapport à la masse totale des ingrédients mis en oeuvre dans la composition.

La présente invention concerne donc une composition pour produit frit contenant de la farine de microalgues et un liquide potable se substituant partiellement mais de préférence totalement aux matières grasses d'origine végétale et/ou animale habituellement utilisées.

Il est à noter, dans la présente invention, que la farine de microalgues vient en substitution d'une partie mais de préférence de la totalité des matières grasses d'origine présentes dans la composition pour produit frit. Ceci permet d'alléger la charge calorique de la composition pour produit frit, mais également d'en modifier le profil lipidique en réduisant notamment l'apport de mauvaises graisses.

Selon l'invention, la farine de microalgue représente au minimum 0,5%, ce pourcentage étant exprimé par rapport à la masse totale des ingrédients mis en oeuvre dans la composition pour produit frit.

Selon l'invention, la composition pour produit frit comprend de 0,5% à 20% de farine de microalgues, préférentiellement de 1% à 15%, plus préférentiellement de 1% à 10%, et de manière encore préférée 1% à 5%, ces pourcentages étant exprimés par rapport à la masse totale des ingrédients mis en oeuvre dans la composition.

Dans un mode de réalisation préféré, la somme des composants consistant en la farine de microalgues et le liquide potable représente au minimum 20, 25, 30, 35, 40, 50%, ces pourcentages étant exprimés par rapport à la masse totale des ingrédients mis en oeuvre dans la composition.

Notamment, la somme des composants consistant en la farine de microalgues et le liquide potable représente au moins 20%, préférentiellement au moins 25%, plus préférentiellement au moins 30% en masse de la totalité des ingrédients utilisés dans la composition pour produit frit.

La farine de microalgues selon l'invention est parfaitement apte à se substituer aux matières grasses traditionnellement utilisées et améliorent très nettement le profil nutritionnel des produits frits qui la contiennent.

La Demanderesse a trouvé de façon surprenante et inattendue que le fait d'utiliser de la farine de microalgues riche en lipides et/ou en fibres dans la composition pour produit frit permet de diminuer la charge calorique de ladite composition en diminuant la quantité de matière grasse utilisée.

Ainsi ladite farine de microalgues riches en lipides et/ou en fibres possède d'une part une propriété de matière grasse de substitution (« fat replacer ») en permettant au produit frit de conserver les caractéristiques sensorielles recherchées tant au niveau de la texture (fondant, moelleux) que du goût, car elle possède une capacité de rétention d'eau importante qui permet de mettre plus d'eau dans la composition pour produit frit. Qui plus est, l'eau rajoutée en plus dans la composition pour produit frit selon l'invention a la particularité de rester au niveau de la composition et donc du produit frit en résultant sans en affecter l'activité de l'eau et la stabilité du produit frit final.

Le fait d'avoir des compositions pour produit frit plus hydratées améliore de façon significative le moelleux sur le produit frit final. Non seulement la matière grasse est diminué mais les qualités organoleptiques sont améliorées. L'intérêt de l'invention est donc multiple.

De plus, le caractère moelleux est conservé au cours du temps. Ainsi la durée de conservation également appelée DLC ou Date Limite de Consommation est augmentée. Ce qui présente à nouveau un avantage certain pour les nombreux industriels de la pâtisserie qui recherchent sans cesse des durées de conservation allongées sans avoir recours à des additifs boudés des consommateurs.

Les algues font partie des premiers organismes apparus sur Terre, et sont définies comme des organismes eucaryotes dépourvus de racines, de tige et de feuille, mais possédant de la chlorophylle ainsi que d'autres pigments accessoires à la photosynthèse productrice d'oxygène. Elles sont bleues, rouges, jaunes, doré et brun ou encore vertes. Elles représentent plus de 90% des végétaux marins et 18% du règne végétal, avec leurs 40 000 à 45 000 espèces. Les algues sont des organismes extrêmement variés tant par leur taille et leur forme que par leur structure cellulaire. Elles vivent en milieu aquatique ou très humide. Elles contiennent de nombreuses vitamines et oligo-éléments, et sont de véritables concentrés d'actifs stimulants et bienfaisants pour la santé et la beauté. Elles ont des vertus anti-inflammatoires, hydratantes, adoucissantes, régénérantes, raffermissantes, anti-âge. Elles possèdent également des caractéristiques "technologiques" qui permettent d'apporter de la texture à un produit alimentaire. En effet, les fameux additifs E400 à E407 ne sont en fait que des composés extraits d'algues, dont on utilise les propriétés épaississantes, gélifiantes, émulsifiantes et stabilisantes.

Parmi les algues, on peut distinguer les macroalgues et les microalgues, notamment algues microscopiques unicellulaires, photosynthétiques ou non, d'origine marine ou non, cultivées notamment pour leurs applications en Bio-carburant ou dans le domaine alimentaire. Par exemple, la spiruline (Arthrospira platensis) est cultivée dans des lagunes ouvertes (en phototrophie) pour une utilisation comme complément alimentaire ou incorporée en petites quantités dans des confiseries ou des boissons (généralement moins de 0,5 % p/p). D'autres microalgues riches en lipides, y compris certaines espèces de Chlorella, sont également très populaires dans les pays asiatiques comme compléments alimentaires (citons les microalgues du genre Crypthecodinium ou Schizochytrium). La production et l'utilisation de farines de microalgues est décrite dans les demandes WO 2010/120923 et WO 2010/045368.

Au sens de la présente invention, le terme « farine de microalgues » doit être compris dans son interprétation la plus large et comme désignant par exemple, une composition comprenant une pluralité de particules de biomasse de microalgues. La biomasse de microalgues est dérivée de cellules de microalgues, qui peuvent être entières ou cassées, ou un mélange de cellules entières et cassées. Il est entendu dans le présent document que la farine de microalgues désigne un produit essentiellement composé de biomasse de microalgues, c'est-à-dire à au moins 90, 95, 99%. Dans un mode de réalisation préféré, la farine de microalgues comprend uniquement de la biomasse de microalgues.

La présente invention est ainsi relative à la biomasse de microalgues propre à la consommation humaine qui est riche en nutriments, notamment en lipides et / ou protéines.

L'invention se rapporte également à une farine de microalgues qui peut être incorporée dans les produits alimentaires dans lesquels la teneur en lipides et / ou en protéines de la farine de microalgues peut se substituer en totalité ou en partie pour les huiles et / ou matières grasses et / ou protéines présentes dans les produits alimentaires conventionnels.

La fraction lipidique de la farine de microalgues, qui peut se composer essentiellement d'huiles monoinsaturées, fournit ainsi des avantages nutritionnels et de santé par rapport aux huiles saturées, hydrogénées et polyinsaturées souvent trouvées dans les produits alimentaires conventionnels.

La fraction protéique de la farine de microalgues qui renferme beaucoup d'acides aminés essentiels au bien-être humain et animal fournit donc également des avantages nutritionnels et de santé intéressants et non négligeables.

Au sens de l'invention, les microalgues considérées sont les espèces qui produisent des huiles appropriées et / ou des lipides et / ou des protéines.

Selon l'invention, la biomasse de microalgues comprend au moins 10% en poids sec de lipides, de préférence au moins 12% et encore plus préférentiellement de 25 à 35% ou plus en poids sec de lipides.

Ainsi, selon la présente invention, l'expression « riche en lipides » doit être interprétée comme se référant à des teneurs d'au moins 10% en poids sec de lipides, de préférence d'au moins 12% en poids sec de lipides et encore plus préférentiellement des teneurs d'au moins 25 à 35% ou plus en poids sec de lipides.

Selon un mode préférentiel de l'invention, la biomasse de microalgues contient au moins 12%, au moins 25%, au moins 35%, au moins 40%, au moins 50%, ou au moins 75% en poids sec de lipides. Par exemple, la biomasse de microalgues comprend entre 45% et 55% en poids sec de lipides.

Selon un mode particulièrement préféré, la biomasse des microalgues contient au moins 40% de lipides, et de préférence au moins 50% de lipides en poids sec.

Selon un autre mode de réalisation de l'invention, la biomasse de microalgues contient au moins 10% en poids sec de protéines, au moins 20%, au moins 30%, au moins 40%, ou au moins 45% en poids sec de protéines.

Dans un mode de réalisation particulier, la biomasse des microalgues contient au moins 35% en poids sec de lipides et au plus 20% en poids sec de protéines.

Ainsi, selon la recette du produit, le boulanger pâtissier pourra choisir d'incorporer à sa recette de composition pour produits frits plutôt une farine de microalgues présentant une teneur élevée en lipides ou plutôt une farine de microalgues algues présentant une teneur élevée en protéines, une farine de microalgues présentant à la fois une teneur élevée en lipides et protéines, ou encore un mélange des deux types de farines de microalgues.

Selon un mode préféré, l'homme du métier choisira une farine de microalgues présentant une teneur élevée en lipides, d'au moins 40% et de préférence d'au moins 50% en poids sec.

Selon un autre mode préférentiel de l'invention, les microalgues appartiennent au genre *Chlorella.*

La chlorelle (ou Chlorella) est une algue unicellulaire verte microscopique ou microalgue d'eau douce apparue sur la terre depuis plus de 3 milliards d'années, appartenant à l'embranchement des Chlorophytes. La chlorelle possède la plus grande concentration en chlorophylle de tous les végétaux, et sa capacité à la photosynthèse est considérable. Depuis sa découverte, la chlorelle n'a cessé de générer un intérêt considérable dans le monde, et aujourd'hui elle est produite à grande échelle pour des utilisations dans des compléments alimentaires et nutritionnels. En effet, la chlorelle contient plus de 60% de protéines qui renferment beaucoup d'acides aminés essentiels au bien-être humain et animal. La chlorelle contient également beaucoup de vitamines (A, Bêta carotène, B1 : thiamine, B2 : riboflavine, B3 : niacine, B5 : acide pantothénique, B6 : pyridoxine, B9 : acide folique, B12 : cobalamine, vitamine C : acide ascorbique, vitamine E : tocophérol, vitamine K : phylloquinone), de lutéine (famille des caroténoïdes, puissant antioxydant), et de minéraux dont le calcium, le fer, le phosphore, le manganèse, le potassium, le cuivre et le zinc. En outre, la chlorelle contient certains acides gras polyinsaturés de type oméga indispensables au bon fonctionnement cardiaque et cérébral et à la prévention de nombreuses maladies comme le cancer, le diabète ou l'obésité.

Les bienfaits rapportés à la consommation de chlorelle sont très nombreux. C'est un complément alimentaire utilisé quotidiennement au Japon par 4 millions de personnes. A tel point que le gouvernement japonais l'a classée comme « aliment d'intérêt national ».

Facultativement, les microalgues utilisées peuvent être choisies, et de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora et Prototheca moriformis.* De manière préférée, les microalgues utilisées selon l'invention appartiennent à l'espèce *Chlorella protothecoides.*

Dans le cadre de l'invention, *Chlorella protothecoides* est choisie en raison de sa composition élevée en lipides.

Dans un mode de réalisation secondaire, *Chlorella protothecoides* est également choisie en raison de sa composition élevée en protéines.

Dans la farine de microalgues, les parois cellulaires des microalgues et/ou les débris cellulaires de ces dernières peuvent optionnellement encapsuler les lipides au moins jusqu'à ce que le produit alimentaire la contenant soit cuit, ce qui augmente la durée de vie des lipides.

La farine de microalgues fournit également d'autres bénéfices, comme des micronutriments, des fibres alimentaires (glucides solubles et insolubles), des phospholipides, des glycoprotéines, des phytostérols, tocophérols, tocotriénols, et du sélénium.

Selon un mode de réalisation de l'invention, la composition pour produit frit comprend une farine de microalgues dont le teneur en fibres totale est d'au moins 15%, et de préférence d'au moins 18% en poids sec de sa composition.

Selon un mode de réalisation de l'invention, les microalgues peuvent être modifiées de manière à réduire la production de pigments, voire à l'inhiber totalement. Par exemple, Chlorella protothecoides peut être modifiée par mutagenèses par UV et/ou chimique de manière à être réduite ou dénuée en pigments.

Il peut en effet être particulièrement intéressant d'avoir des microalgues dépourvues de pigment de façon à éviter l'obtention d'une couleur verte plus ou moins marquée dans les produits de cuisson dans lesquels la farine de microalgues est employée.

Comme les microalgues sont destinées à la production de farine destinées à des formulations alimentaires, selon un mode préféré de réalisation de l'invention, les microalgues ne subissent aucune modification génétique comme par exemple mutagénèse, transgénèse, génie génétique et/ou chimique. Ainsi, les microalgues n'ont pas subi de modifications de leur génome par quelque technique de biologie moléculaire que ce soit.

Selon ce mode préféré, les algues destinées à la production de la farine de microalgues possèdent le statut GRAS. Le concept GRAS (Generally Recognized As Safe) créé en 1958 par la Food and Drug Administration (FDA) permet la régulation de substances ou extraits ajoutés aux aliments et qui sont considérés comme sans danger par un panel d'expert.

Les conditions appropriées de culture à utiliser sont notamment décrites dans l'article d'Ikuro Shihira-Ishikawa et Eiji Hase, « Nutritional Control of Cell Pigmentation in Chlorella protothecoides with special reference to the degeneration of chloroplast induced by glucose », Plant and Cell Physiology, 5, 1964.

Cet article décrit notamment que tous les grades de couleur peuvent être produits par Chlorella protothecoides (incolore, jaune, vert jaunâtre et vert), en faisant varier les sources et les ratios d'azote et de carbone. En particulier, des cellules « délavées » et « incolores » sont obtenues en utilisant des milieux de culture riches en glucose et pauvres en azote. La distinction entre cellules incolores et cellules jaunes est faite dans cet article. De plus, les cellules délavées cultivées en excès de glucose et en azote limité possèdent de fort taux de croissance. De plus, ces cellules contiennent de fortes quantités de lipides.

D'autres articles tel que celui de Han Xu, Xiaoling Miao, Qingyu Wu , « High quality biodiesel production from a microalga Chlorella protothecoides by heterotrophic growth in fermenters », Journal of Biotechnoloy, 126, (2006), 499-507 décrivent que des conditions de culture hétérotrophiques, c'est-à-dire en absence de lumière permettent d'obtenir une biomasse élevée avec une teneur élevée en lipides dans les cellules de microalgues.

Les milieux de croissance solides et liquides sont généralement disponibles dans la littérature, et les recommandations pour la préparation des milieux particuliers qui conviennent à une grande variété de souches de microorganismes peuvent être trouvées, par exemple, en ligne à www.utex.org/, un site maintenu par l'Université du Texas à Austin pour sa collection de culture d'algues (UTEX).

Au vu de ses connaissances générales et de l'état de l'art précité, l'homme du métier chargé de cultiver les cellules de microalgues sera tout à fait capable d'adapter les conditions de culture afin d'obtenir une biomasse nombreuse, riche en protéines et/ou en lipides et dépourvue soit en totalité soit de façon atténuée de pigments chlorophylliens.

Selon la présente invention, les microalgues sont cultivées en milieu liquide pour produire la biomasse en tant que telle.

Selon la présente invention, les microalgues sont cultivées dans un milieu contenant une source de carbone et une source d'azote, soit en présence de lumière, soit en absence de lumière.

Selon un mode préféré de l'invention, les microalgues sont cultivées dans un milieu contenant une source de carbone et une source d'azote en absence de lumière (conditions hétérotrophiques).

La production de biomasse est réalisée en fermenteurs (ou bioréacteurs). Les exemples spécifiques de bioréacteurs, les conditions de culture, et la croissance hétérotrophe et les méthodes de propagation peuvent être combinés de toute manière appropriée pour améliorer l'efficacité de la croissance microbienne et des lipides et / ou de production de protéines.

Pour préparer la biomasse pour une utilisation dans des compositions alimentaires, la biomasse obtenue en fin de fermentation est concentrée ou récoltée, du milieu de fermentation. Au moment de la récolte de la biomasse de microalgues du milieu de fermentation, la biomasse comprend des cellules intactes pour l'essentiel en suspension dans un milieu de culture aqueux.

Pour concentrer la biomasse, on procède alors à une étape de séparation solide-liquide, par filtration, par centrifugation ou par tout moyen connu par ailleurs de l'homme du métier.

Après concentration, la biomasse de microalgues peut être traitée afin de produire des gâteaux emballés sous vide, des paillettes d'algues, des homogénats d'algues, de la poudre d'algues, de la farine d'algues, ou de l'huile d'algues.

On procède également au séchage de la biomasse de microalgues pour faciliter le traitement ultérieur ou pour une utilisation de la biomasse dans ses différentes applications, notamment alimentaires.

Différentes textures et saveurs peuvent être conférées à des produits alimentaires, selon que la biomasse algale est séchée, et si oui, en fonction de la méthode de séchage mise en oeuvre. Se référer aux brevets US 6.607.900 et US 6.372.460 pour des exemples.

Selon la présente invention, la farine de microalgues peut être préparée à partir de la biomasse de microalgues concentrée qui a été mécaniquement lysée et homogénéisée, l'homogénat étant ensuite atomisé ou flash-séché.

Selon un mode de réalisation de l'invention, les cellules utilisées pour la production de farine de microalgues sont lysées pour libérer leur huile ou lipides. Les parois cellulaires et les composants intracellulaires sont broyés ou réduits, par exemple grâce à un homogénéisateur, en particules ou débris cellulaires non agglomérés. Selon un mode préférentiel de l'invention, les particules résultantes possèdent une taille moyenne inférieure à 500 µm, 100 µm ou même 10 µm ou moins.

Selon un autre mode de réalisation de l'invention, les cellules lysées peuvent également être séchées.

Par exemple, un disrupteur à pression peut être utilisé pour pomper une suspension contenant les cellules à travers un orifice restreint pour lyser les cellules. Une pression élevée (jusqu'à 1500 bars) est appliquée, suivie d'une expansion instantanée à travers une buse. Le cassage des cellules peut être réalisé par trois mécanismes différents : empiètement sur la vanne, cisaillement élevé du liquide dans l'orifice, et chute de pression soudaine en sortie, provoquant une explosion de la cellule.

La méthode libère des molécules intracellulaires.

Un homogénéisateur NIRO (GEA NIRO SOAVI) (ou tout autre homogénéisateur haute pression) peut être utilisé pour casser des cellules.

Ce traitement de la biomasse algale sous haute pression (environ 1500 bars) lyse généralement plus de 90 % des cellules et réduit la taille des particules à moins de 5 microns.

Selon un mode de réalisation de l'invention, la pression appliquée est de 900 bars à 1200 bars. De manière préférentielle la pression appliquée est de 1100 bars.

Selon un autre mode de réalisation, et afin d'augmenter le pourcentage de cellules lysées, la biomasse de microalgues peut subir un double traitement à haute pression, voire plus (triple traitement, ..).

Selon un mode préféré, une double homogénéisation est utilisée afin d'augmenter le pourcentage de cellules lysées et obtenir un taux supérieur à 50%, supérieur à 75% ou supérieur à 90%. Un pourcentage de cellules lysées pouvant aller jusqu'à environ 95% a été observé grâce à ce double traitement. Ainsi, dans un exemple de réalisation, la farine de microalgues dans la composition selon l'invention comprend entre 50% et 95%, préférentiellement entre 60% et 95%, encore plus préférentiellement entre 75% et 90% en poids de cellules lysées.

La lyse des cellules de microalgues est optionnelle mais préférée lorsqu'une farine riche en lipides (e.g ; supérieure à 10%) est souhaitée.

Selon un premier mode de réalisation de l'invention, une lyse partielle est souhaitée, c'est-à-dire que la farine de microalgues est sous la forme de cellules partiellement lysées et contient de 25% à 75%, de 30% à 70%, de 35% à 65%, de 40% à 60%, de 45% à 55% de cellules lysées.

Selon un second mode de réalisation particulièrement préféré de l'invention, une lyse maximale voire totale est souhaitée, c'est-à-dire que la farine de microalgues est sous la forme de cellules fortement voire totalement lysées et contient 85% ou plus de cellules lysées, notamment, 86%, 87%, 88%, 89%, 90%, 91%, 92%, 93%, 94%, 95%, 96%, 97%, 98% ou 99% et de préférence plus de 90%.

Ainsi selon ce mode préféré de l'invention, la composition pour produit frit comprend une farine de microalgues dont les cellules ont été lysées totalement par l'un des traitements précités et parfaitement connu par l'homme du métier.

Ainsi dans la présente invention, la farine de microalgue est susceptible d'être sous une forme non broyée jusqu'à une forme extrêmement broyée avec des taux de broyage supérieurs à 95%. Des exemples spécifiques portent sur des farines de microalgues présentant des taux de broyage d'au moins 50%, 75%, 85% ou 95 % de lyse des cellules, de préférence de 85%, 86%, 87%, 88%, 89%, 90%, 91%, 92%, 93%, 94%, ou 95 %.

Dans un autre mode de réalisation de l'invention, une farine de microalgues riche en protéines est produite. Cette farine de microalgues riches en protéines peut être sous la forme de cellules non lysées (cellules intactes non lysées et non broyées).

De manière alternative, un broyeur à billes est plutôt utilisé. Dans ce type de broyeur, les cellules sont agitées en suspension avec de petites particules abrasives. Le cassage des cellules est provoqué par les forces de cisaillement, le broyage entre les billes, et les collisions avec des billes. En fait, ces billes cassent les cellules pour en libérer le contenu cellulaire. La description d'un broyeur à billes approprié est par exemple faite dans le brevet US 5.330.913.

On obtient une suspension de particules, optionnellement de plus petite taille que les cellules d'origine sous la forme d'une émulsion « huile dans eau ». Cette émulsion peut ensuite être atomisée et l'eau est éliminée, laissant une poudre sèche contenant les débris cellulaires et les lipides. Après séchage, la teneur en eau ou l'humidité de la poudre est généralement inférieure à 10 %, préférentiellement inférieure à 5% et de façon plus préférée inférieure à 3% en poids.

Cependant, la poudre obtenue renfermant de l'huile à une teneur de 10%, 25% voire 50 % en poids de la poudre sèche, on peut déplorer l'obtention d'une poudre sèche d'aspect collante, qui s'écoule difficilement. Différents agents d'écoulement (y compris les produits dérivés de la silice) peuvent alors être ajoutés. Il peut être également rencontré des problèmes de dispersibilité dans l'eau des farines de biomasse séchées, présentant alors de moins bonnes propriétés de mouillabilité.

La société Demanderesse a mis au point des granules de farine de microalgues présentant une distribution granulométrique particulière, des propriétés d'écoulement et de mouillabilité remarquables. Notamment, ces granules permettent de stabiliser la farine de microalgues, et de permettre leur incorporation aisée, à grande échelle, dans des produits alimentaires qui doivent rester savoureux et nutritifs. Le procédé de préparation desdits granules est parfaitement décrit dans les deux demandes de brevet EP 2724625 et EP 2777400 dont la Demanderesse est également titulaire et qui sont incorporés dans la présente demande par référence.

Comme décrit dans les deux demandes de brevet précitées et selon un mode de réalisation de l'invention, la farine de microalgues utilisée dans la composition pour produit fritest sous la forme de granules de farine de microalgues présentant une ou plusieurs des caractéristiques suivantes, de préférence les trois :
- une distribution granulométrique monomodale, mesurée sur un granulomètre laser LS de marque COULTERO, comprise entre 2 et 400 µm, centrée sur un diamètre de particules (D mode) entre 5 et 15µm,
- des notes d'écoulement, déterminées selon un test A, comprises entre 0,5 et 60 % en poids pour le refus à 2.000 µm, comprises entre 0,5 et 60 % en poids pour le refus à 1.400 µm et comprises entre et 0,5 et 95 % en poids pour le refus à 800 µm,
- un degré de mouillabilité, exprimé selon un test B, par la hauteur du produit décanté dans un bécher à une valeur comprise entre 0 et 4 cm, de préférence entre 0 et 2 cm, et plus préférentiellement entre 0 et 0,5 cm.

Le test A consiste à mesurer le degré de cohésion des granules de farine de microalgues. Le test A mis au point par la société Demanderesse est plus précisément décrit dans la demande antérieure EP 2 724 625 et plus particulièrement aux paragraphes [0066] - [0070] de la publication, qui sont intégrés par référence.

Le test B consiste à mesurer le degré de mouillabilité des granules de farine de microalgues. Le test B mis au point par la société Demanderesse est plus particulièrement décrit dans la demande antérieure EP 2 724 625 et plus particulièrement aux paragraphes [0086] - [0089] de la publication, qui sont intégrés par référence.

L'invention concerne donc également une composition pour produit frit comprenant de la farine, de la levure, de la farine de microalgue sous la forme de granules et un liquide potable.

Selon l'invention, la composition pour produit frit comprend également de la levure. Cette dernière est nécessaire pour faire gonfler la pâte et lui donner une structure souple et légère avec une texture aérée sur le produit frit final.

Selon l'invention la levure peut être soit de la levure chimique soit de la levure de boulangerie constituée de microorganismes soit un mélange des deux.

Selon l'invention, la composition pour produits frits est caractérisée en ce qu'elle comprend jusqu'à 2%, de préférence moins de 1% de levure chimique, ces pourcentages étant exprimés par rapport à la masse totale des ingrédients mis en oeuvre dans la composition. Selon l'invention, la composition pour produit frit comprend entre 0.1% et 1%, préférentiellement entre 0,2% et 0,8%, plus préférentiellement entre 0,3% et 0,7%, et encore plus préférentiellement entre 0.4% et 0.6% de levure chimique, ces pourcentages étant exprimés par rapport à la masse totale des ingrédients mis en oeuvre dans la composition.

Selon un autre mode de réalisation de l'invention, la composition pour produits frits est caractérisée en ce qu'elle comprend jusqu'à 10%, de préférence moins de 7%, et encore plus préférentiellement moins de 5% de levure de boulangerie, ces pourcentages étant exprimés par rapport à la masse totale des ingrédients mis en oeuvre dans la composition.

Selon l'invention, la composition pour produit frit comprend entre 1% et 10%, notamment entre 1 % et 7%, entre 1% et 4%, et de préférence entre 2 et 3,7% de levure de boulangerie, ces pourcentages étant exprimés par rapport à la masse totale des ingrédients mis en oeuvre dans la composition.

Selon l'invention, la levure chimique (ou poudre à lever) est un mélange composé essentiellement d'un agent basique (tel que le bicarbonate de sodium), un agent acide (acide tartrique, pyrophosphate de sodium) et un agent stabilisant (tel que l'amidon), se présentant sous forme de poudre blanche et servant à faire gonfler les pâtes. Contrairement à la levure de boulanger, qui agit par la fermentation de micro-organismes vivants, la levure chimique fait seulement intervenir des réactions chimiques de type acide-base. Tant que la poudre reste sèche, la réaction ne démarre pas. Lorsqu'elle est humidifiée, l'acide réagit avec le bicarbonate de sodium et un dégagement de dioxyde de carbone se produit, ce qui fait gonfler la pâte. Il faut alors la cuire sans tarder.

La levure de boulangerie (*Saccharomyces cerevisiae*) est une levure, c'est-à-dire un champignon unicellulaire microscopique. A la différence de la levure chimique, la levure de boulangerie est un produit vivant. Ses cellules possèdent la propriété de transformer les sucres naturellement présents dans la farine en alcool et en dioxyde de carbone. En l'absence de dioxygène, le glucose est dégradé en éthanol (alcool) et dioxyde de carbone par la levure selon la réaction de fermentation alcoolique. Le gaz ainsi fabriqué lors de la cuisson fait gonfler la pâte. L'éthanol formé s'évapore lors de la cuisson.

Selon l'invention, la levure de boulangerie peut être choisie parmi la levure pressée, la levure émiettée, la levure liquide, la levure sèche active à réhydrater préalablement, la levure sèche à humidité intermédiaire surgelée et leurs mélanges quelconques.

Selon l'invention, la composition pour produit frit peut être aromatisée grâce à l'ajout d'au moins un arôme (c'est-à-dire d'un ou plusieurs arômes).

Dans la présente invention, l'appellation « arôme » désigne toutes substances non destinées à être consommées en l'état, qui sont ajoutés aux denrées alimentaires pour leur conférer une odeur et/ou un goût ou modifier ceux-ci. Elles sont issues ou constituées des catégories suivantes: substances aromatisantes, préparations aromatisantes, arômes obtenus par traitement thermique, arômes de fumée, précurseurs d'arôme ou autres arômes ou leurs mélanges.

Les substances aromatisantes sont des substances chimiques définies, ce qui inclut les substances aromatisantes obtenues par synthèse chimique ou isolées par des procédés chimiques, et les substances aromatisantes naturelles. Les préparations aromatisantes sont des arômes, autres que des substances chimiques définies, qui sont obtenues par des procédés physiques, enzymatiques ou microbiologiques appropriés, à partir de matières d'origine végétale, animale ou microbiologique prises en l'état ou après leur transformation pour la consommation humaine. Les précurseurs d'arôme tels que les hydrates de carbone, les oligopeptides et les acides aminés confèrent une flaveur aux denrées alimentaires par des réactions chimiques qui se produisent pendant la transformation de ces denrées.

Selon un mode préférentiel, l'arôme utilisé peut être choisi parmi l'arôme vanille, cannelle, chocolat, fruit, fruits secs comme notamment l'arôme noisette, amande, cacahuète, noix de cajou, noix de pécan, pistaches...

Selon un autre mode de réalisation secondaire, l'arôme utilisé peut être choisi parmi les arômes salés de type jambon, jambon fumé, foie gras, champignons, pizza, bacon, ...

Selon un mode de réalisation de l'invention, la composition pour produit frit comprend entre 0,5% et 5%, préférentiellement entre 0.6% et 4%, plus préférentiellement entre 0,8% et 3%, et encore plus préférentiellement entre 1% et 2.5% d'arômes, ces pourcentages étant exprimés par rapport à la masse totale des ingrédients mis en oeuvre dans la recette.

Selon un autre mode de réalisation, la composition pour produit frit selon l'invention peut également contenir des améliorants permettant de renforcer le côté moelleux de la pâte.

L'invention a donc également pour objet une composition pour produit frit comprenant
- entre 0.5% et 20% de farine de microalgues, préférentiellement entre 1% et 5%,
- entre 5% et 75% d'un liquide potable, et notamment de l'eau,
- entre 10% et 80% de farine, et
- entre 0.1% et 1% de levure chimique ou entre 1% et 10% de levure de boulangerie,
ces pourcentages étant exprimés par rapport à la masse totale des ingrédients de la composition. La composition peut en outre comporter entre 0,5% et 30% d'oeufs ou ovoproduits, et/ou entre 0.5% et 5% d'arômes et/ou entre 0.5% et 20% de matière sucrante et/ou entre 0.5% et 30% de dérivés du lait.

Dans un exemple particulier, la composition pour produit frit comprend
- environ 1.5% de farine de microalgues,
- environ 32% d'un liquide potable, et notamment de l'eau,
- environ 52% de farine,
- environ 3,5% d'oeufs ou ovoproduits,
- environ 0,5% de levure chimique,
- environ 6.5% de matière sucrante,
- environ 2% de dérivé du lait,
- environ 2% d'arôme.

Dans un autre exemple particulier, la composition pour produit frit comprend
- environ 4% de farine de microalgues,
- environ 38% d'un liquide potable, et notamment de l'eau,
- environ 50% de farine,
- environ 5% d'oeufs ou ovoproduits,
- environ 2% de levure de boulanger,
- environ 1% d'arôme.

Dans un mode préférentiel, l'invention est destinée aux produits alimentaires des domaines de la biscuiterie, pâtisserie et viennoiserie.

Dans la présente invention, les termes « boulangerie », « pâtisseries », « produits frits » doivent être interprétés de manière large, comme se référant en général au domaine de la production de produits frits à partir de compositions de pâtes fermentées à base d'amidon, ainsi qu'aux domaines de la boulangerie et de la viennoiserie.

Un autre aspect de l'invention concerne également un procédé de préparation d'un produit frit comprenant une composition selon l'invention
Selon l'invention, le procédé de préparation d'un produit frit comprend le mélange de farine, d'oeufs ou ovoproduits, de levure et de farine de microalgues à un liquide potable, avant cuisson.

Dans une mise en oeuvre particulière, le procédé comprend les étapes suivantes:
- une étape de mélange de la farine, des oeufs ou ovoproduits, de la levure et de la farine de microalgues à un liquide potable,
- une étape de fermentation de ce mélange,
- une étape de mise en forme dudit mélange en boule(s) de pâte,
- une étape de cuisson de la ou desdites boule(s) de pâte dans un bain d'huile chaude entre 150°C et 200°C pendant une durée allant de 30 secondes à 3 minutes pour chaque face.

Dans un mode préférentiel de réalisation du procédé selon l'invention, le liquide potable est de l'eau.

La cuisson de la composition pour produit frit selon l'invention dans un bain d'huile chaude est une étape critique car le produit frit absorbe une quantité non négligeable d'huile de friture, ce qui augmente considérablement sa charge calorique d'une part, mais également sa teneur en lipides de type trans, qui comme expliqués ci-dessus sont néfastes pour la santé car augmentant le risque de maladies cardiovasculaires.

Pourtant cette étape de friture reste incontournable car elle permet de conférer au produit fini sa texture particulière croustillante à l'extérieur et moelleuse à l'intérieur.

La Demanderesse a trouvé de façon surprenante et inattendue que le fait d'utiliser de la farine de microalgues riche en lipides et en fibres dans la composition pour produit frit permet d'une part de diminuer la charge calorique de ladite composition en diminuant la quantité de matière grasse utilisée, mais permet également de diminuer la quantité d'huile absorbé par le produit frit pendant sa cuisson dans le bain d'huile.

Ainsi de manière surprenante et inattendue, la Demanderesse en cherchant à diminuer la teneur en matières grasses dans des compositions pour produit frit en y substituant la matière grasse traditionnellement utilisée par une farine de microalgues, et de préférence une farine de microalgues riches en lipides et/ou en fibres, a également constaté que les compositions pour produit frit selon l'invention présentaient une reprise en matière grasse liée à leur cuisson dans un bain d'huile moins importante que les compositions pour produit frit traditionnelles.

La présente invention concerne donc également un procédé de préparation pour produit frit comprenant les étapes précédemment décrites et permettant d'obtenir des produits frits possédant une teneur en matières grasses totales réduite d'au moins 30%, de préférence, d'au moins 40% et encore plus préférentiellement d'au moins 45% par rapport au produit frit obtenu selon une composition classique.

Selon un mode particulièrement intéressant, la présente invention concerne donc également un procédé de préparation pour produit frit comprenant les étapes précédemment décrites et permettant d'obtenir des produits frits possédant une teneur en matières grasses totales réduite d'au moins 50% par rapport au produit frit obtenu selon une composition classique.

Dans la présente invention, la teneur en matières grasses totales mesurée sur les produits frits inclut à la fois les matières grasses utilisées dans la composition pour produit frit mais également celles apportées par la friture dans un bain d'huile chaud et/ou selon les autres moyens décrits précédemment.

L'intérêt de l'invention est donc multiple.

Un dernier aspect de l'invention concerne aussi un produit frit comprenant de la farine de microalgues et dont la teneur en matière grasse est réduite d'au moins 30%, de préférence, 40% et encore plus préférentiellement de 45% par rapport au produit frit obtenu selon une composition classique, exempte de farine de microalgues.

Selon ce dernier aspect, la présente invention concerne également un produit frit comprenant de la farine de microalgues et dont la teneur en matière grasse est réduite d'au moins 50% par rapport au produit frit obtenu selon une composition classique.

Ledit produit frit selon l'invention est obtenu en mettant en oeuvre une composition pour produit frit selon l'invention et/ou en utilisant le procédé de préparation du produit frit également selon l'invention.

Du fait de la réduction significative de la teneur finale en matières grasses totales, le produit frit selon l'invention possède un profil nutritionnel beaucoup plus intéressant et sain que le profil nutritionnel d'un produit frit préparé selon une recette classique, exempte de farine de microalgues.

Comme précisé précédemment dans la présente invention, la dénomination « produit frit » utilisée doit être comprise dans son interprétation la plus large et comme désignant un produit constitué d'un mélange à minima de farine, de levure et de matière grasse, et dont le mélange est ensuite mis à frire par petite quantité de pâte préférentiellement dans un bain d'huile chaude entre 150°C et 250°C pendant une durée allant de 2 secondes à 3 minutes pour chaque face. Ledit produit frit peut également être frit après avoir été vaporisé d'huile, déposé dans un plat et soumis à des radiations infrarouge ou encore être frit après avoir été vaporisé d'huile, déposé dans un plat et soumis à une cuisson dans un four.

Ainsi, la dénomination « produit frit » désigne particulièrement les produits de type beignets, donuts, churros, chichis, croustillons, bugnes, tempuras, etc..

Selon un mode de réalisation préférentiel les produits frits selon l'invention peuvent être sucrés ou salés. Ils peuvent être natures ou bien fourrés de confiture, chocolat, crème pâtissière, chantilly, foie gras, mousses salées, etc..

Selon un mode préféré de l'invention, les produits frits sont sucrés et contiennent de préférence du saccharose, de l'allulose et/ou leurs mélanges quelconques.

Selon un autre mode de réalisation de l'invention, ils peuvent également être saupoudrés de sucre, glacés, glacés au sucre fondu, recouverts d'un nappage sucré et aromatisé, etc..

Selon un autre mode de réalisation de l'invention, les produits frits peuvent également être fourrés d'une crème pâtissière, de chantilly salée ou sucrée et/ou aromatisée, d'une ganache, d'un fourrage gras.

La ganache est une préparation épaisse de chocolat servant à garnir une confiserie ou une pâtisserie. Dans sa forme la plus simple, la ganache est un mélange de crème (ou parfois de lait ou de beurre, voire d'un mélange des trois) et de chocolat, généralement en quantité environ égales.

Les fourrages gras au lait sont tous les mélanges obtenus à partir de sucre et de matières grasses végétales et/ou animales, qui contiennent traditionnellement des protéines laitières et qui sont destinés à être utilisés comme garniture de remplissage en confiserie, pâtisserie, boulangerie, biscuiterie et tout autre domaine alimentaire. De tels exemples sont par exemple des fourrages gras pralinés, des fourrages gras fantaisie « chocolat ».

Enfin, l'invention porte également sur l'utilisation d'une farine de microalgues comme matière grasse de substitution (« fat replacer ») dans la préparation d'une composition pour produit frit.

L'invention sera encore mieux comprise à la lecture des exemples qui suivent, lesquels se veulent illustratifs en faisant seulement état de certains modes de réalisation et de certaines propriétés avantageuses selon l'invention, et non limitatifs.

### EXEMPLES

### Exemple 1 : Production de la farine de microalgues

Une souche de *Chlorella protothecoides* (référence UTEX 250), est cultivée dans un fermenteur et selon des techniques connues de l'homme du métier, de façon à ce qu'elle ne produise pas de pigment chlorophyllien. La biomasse résultante est ensuite concentrée de façon à obtenir une concentration finale en cellules de microalgues de 150g/l.

Les cellules sont éventuellement désactivées par traitement thermique à travers une zone HTST (Haute Température / Temps Court) à 85°C pendait 1 minute.

Pour la suite des opérations, la température peut être maintenue sous 8-10°C.

Puis la biomasse lavée est broyée à l'aide d'un broyeur à billes pouvant être de type « bead mill » et plusieurs taux de broyage, notamment de lyse sont alors recherchés : 50% de broyage et 85% de broyage.

Dans un des modes de réalisation, aucun broyage n'est appliqué et le taux de broyage est ainsi nul.

La biomasse ainsi générée et éventuellement broyée peut ensuite être pasteurisée sur une zone HTST (1 minute à 70-80°C) et homogénéisée souspression dans un homogénéisateur GAUVIN à deux étages (250 bars au 1er étage / 50 bars au second) après ajustement du pH à 7 à la potasse.

Trois lots de farine de microalgues sont ainsi obtenus :
- lot 0% : aucun broyage n'est appliqué ;
- lot 50% : le taux de lyse des cellules après broyage est de 50% ;
- lot 85% : le taux de lyse des cellules après broyage est de 85% ;

Selon les conditions de culture appliquées, la teneur lipidique de la biomasse de microalgues est supérieure à 35%, et la teneur en protéines inférieure à 20%.

Le lot présentant un taux de broyage de 85% présente une teneur lipidique de la biomasse de microalgues comprise entre 45 % et 55%.

### Exemple 2 : Séchage de l'émulsion « huile dans eau » homogénéisée de farine de microalgues

On procède au séchage des trois lots de biomasse obtenue dans l'exemple 1 dans un dispositif FILTERMAT, pour obtenir les granules de farine de microalgues.

Le procédé d'atomisation consiste à pulvériser la suspension homogénéisée à haute pression dans un dispositif de type FILTERMAT commercialisé par la société GEA/NIRO, muni d'une buse d'injection haute pression de type DELAVAN, dans les conditions suivantes :
- la pression est réglée de 160 à 170 bars,
- température d'entrée d'atomisation : 180°C à 200°C,
- température de sortie : 60°C à 80°C,
- température d'entrée de la zone de séchage : 60°C à 90°C,
- température de sortie : 65°C,
- température d'entrée de la zone de refroidissement : 10°C à 20°C

La poudre arrive alors sur la bande avec une humidité résiduelle comprise entre 2 et 4 %.

En sortie de bande : les granules de farines de microalgues présentent une humidité résiduelle comprise entre 1 et 3 %, de l'ordre de 2 %.

### Exemple 3 : Compositions pour produits frits et produits frits de type donuts allégés en matières grasses selon l'invention

Des donuts allégés en matière grasse ont été préparés selon l'invention à partir de compositions pour produit frit contenant de la farine de microalgues.

Les essais ont été reproduits deux fois afin de démontrer la reproductibilité.

C'est le lot broyé à 85% de l'exemple 1 qui a été utilisé dans lesdites recettes.

La substitution de la matière grasse est telle que décrite ci-dessus dans la description : 100 parties de matière grasse habituellement utilisée d'origine végétale et/ou animale sont remplacées par un mélange de 25 parties de farine de microalgues et 75 parties d'eau.

Les recettes sont données dans le tableau 1 ci-dessous.

Il n'y a pas eu de modifications dans la recette au niveau du procédé de préparation.

### 3.1. Compositions : Tableau 1

| | **TEMOIN** | | **ESSAI** | |
|---|---|---|---|---|
| | **g** | **%** | **g** | **%** |
| Farine de blé | 1000,0 | 48,8 | 1000,0 | 48,8 |
| Gluten | 50 | 2,4 | 50 | 2,4 |
| Saccharose (sucrose) | 125,0 | 6,1 | 125 | 6,1 |
| Farine de microalgues | - | - | 25 | 1,2 |
| Poudre d'oeufs entiers | 50,0 | 2,4 | 50,0 | 2,4 |
| Poudre de lait écrémé | 30,0 | 1,5 | 30,0 | 1,5 |
| Levure chimique (poudre à lever) *Volcano* | 7,5 | 0,4 | 7,5 | 0,4 |
| Acide ascorbique | 0,2 | 0,0 | 0,2 | 0,0 |
| Sel | 10,0 | 0,5 | 10 | 0,5 |
| Améliorants de panification | 7,7 | 0,4 | 7,7 | 0,4 |
| Arôme vanille | 20,0 | 1 | 20,0 | 1 |
| Levure fraîche pressée | 70,0 | 3,4 | 70,0 | 3,4 |
| Matières grasses BISCUITINE 500 | 100 | 4,9 | - | - |
| Eau à 32°C | 580,0 | 28,3 | 655,00 | 31,9 |
| | **2050,4** | **100** | **2050,4** | **100** |

La levure chimique Volcano est commercialisée par la société Puratos, industrialaan 25, 1702 Groot-Bijgaarden, Belgique.

L'améliorant utilisé est de type Nutrisoft 55: émulsifiant de type monoglycéride. Cet améliorant est commercialisé par la société BASF Chemtrade GmbH, Burgbernheim, Allemagne.

La matière grasse de type BISCUITINE 500 est un mélange de graisses végétales fractionnées et non-hydrogénées commercialisé par la société LODERS CROKLAAN BV, également appelée « shortening ».

### 3.2. Protocole de préparation des compositions pour produit frit selon l'invention

- Introduire les différents ingrédients dans le pétrin.
- Pétrissage pendant 2 minutes en vitesse 1, puis pendant 8 minutes en vitesse 2.
- Pointage pendant 10 minutes.
- Découpage, pesée (pâtons de 40gr) et façonnage en boules.
- Apprêt ou pousse en étuve à 49°C, 80% HR pendant30 minutes.

### 3.3. Protocole de préparation des produits frits selon l'invention

- Cuisson des pâtons préalablement obtenus par friture dans une huile à 180°C pendant 1m30s environ sur chaque face.

Le donut est retourné lorsque la surface est bien dorée.

### 3.4. Valeurs nutritionnelles indicatives des compositions pour produit frit avant cuisson par friture

Toutes les valeurs sont données en poids pour 100 gr de composition pour produit frit. Elles sont calculées de façon théorique en fonction de la quantité de chaque ingrédient utilisé et de sa valeur nutritionnelle standard.

**Tableau 2 :**

| | **Contrôle** | **ESSAI** |
|---|---|---|
| Calories (kCal/kJ) | 279 kCal / 1166 kJ | 243 kCal / 1015 kJ |
| Protéines | 10 | 10,1 |
| **Matières grasses** | **7,0** | **2,7** |
| Carbohydrates | 44,1 | 44,3 |
| *...parmi lesquels DP1,2* | 7,1 | 7,1 |
| Fibres | 1,3 | 1,5 |
| ...*fibres insolubles* | *1,2* | *1,3* |
| ...*fibre soluble* | *0,0* | *0,2* |
| **Réduction de la matière grasse** | | **61%** |
| **Réduction calorique** | | **12,9%** |

Ainsi la recette de composition pour produits frits comprenant de la farine de microalgues, permet une réduction de 61% de la matière grasse dans la composition avant friture, ce qui est très significativement moins que la teneur en matière grasse de la composition témoin.

L'intérêt de l'invention sur la réduction des matières grasses dans des compositions pour produits frits grâce à de la farine de microalgues comme substitut est par conséquent démontré.

### Analyse des produits frits obtenus après cuisson

Deux séries d'essais ont été réalisées afin de confirmer la reproductibilité de l'invention.

**Tableau 3 : Première série d'essais**

| | **Contrôle** | **ESSAI 1** |
|---|---|---|
| Matière Sèche % | 71,9 | 67,5 |
| **Matières grasses** | **19** | **9** |
| **Réduction de la matière grasse** | **53%** | |

L'analyse de la teneur en matière grasses sur les donuts après friture a été réalisée par une méthode permettant de doser les lipides totaux, méthode bien connue de l'homme du métier et également connue sous le nom de méthode de Soxhlet.

Les lipides sont insolubles dans l'eau et très solubles dans les solvants organiques, tel que l'hexane. La plupart des méthodes de dosage des lipides exploitent ces propriétés physiques pour extraire les lipides des aliments dans le but de mesurer leur concentration. La méthode Soxhlet est la méthode de référence utilisée pour la détermination de la matière grasse dans les aliments. C'est une méthode gravimétrique, puisqu'on pèse l'échantillon au début et la matière grasse à la fin de l'extraction.

Le principe est simple : une hydrolyse du produit est réalisée par de l'acide chlorhydrique à chaud, puis une étape de précipitation des lipides totaux est effectuée par refroidissement.

Les lipides sont ensuite séparés par filtration, séchés et isolés par une extraction à l'hexane avant d'être quantifiés par analyse gravimétrique.

**Tableau 4 :Deuxième série d'essais**

| | **Contrôle** | **ESSAI 2** |
|---|---|---|
| Matière Sèche % | 70,2 | 66,8 |
| **Matières grasses** | **13,9** | **6,8** |
| **Réduction de la matière grasse** | **51%** | |

Les analyses des taux de matières grasses dans le produit fini montre des résultats un peu différents en fonction des deux séries d'essais, mais le taux de réduction des matières grasses obtenu dans les donuts contenant de la farine de microalgues reste le même : les deux séries permettent d'obtenir une réduction sur le produit frit de 50% sur la matière grasse. Très exactement 53% et 51% de réduction respectivement pour la première et la deuxième série d'essais.

La masse des produits a également été déterminée avant et après cuisson, et les donuts contenant de la farine de microalgues sont plus légers que les donuts témoins après leur friture. Il semble donc qu'ils perdent plus d'eau qu'ils ne reprennent d'huile au cours de la friture, contrairement aux donuts témoins qui ont repris plus d'huile après la cuisson. Ce qui est très surprenant puisque normalement dans ce genre de produits l'eau perdue est remplacée par une reprise en huile plus importante.

Ainsi l'utilisation de farine de microalgues dans des produits frits et en remplacement de la matière grasse traditionnellement utilisée va à l'encontre des préjugés de l'art antérieur.

Ainsi l'intérêt de l'invention est également démontré par une reprise en huile plus faible des donuts contenant de la farine de microalgues lors de l'étape de cuisson par friture.

Et cette diminution du taux d'absorption d'huile pourrait également avoir un impact positif sur le coût de production des donuts selon l'invention.

### Analyse et comparaison de la teneur en matières grasses sur la composition pour produits frits et sur les produits frits

| Tableau 5 | **Contrôle** | **ESSAI** |
|---|---|---|
| Matière grasse sur composition avant cuisson (%) | 7 | 2,7 |
| MG sur produit cuit (1^{ère} série d'essais) (%) | 19 | 9 |
| MG sur produit cuit (2^{ème} série d'essais) (%) | 13,9 | 6,8 |
| | | |
| Différence MG entre produit cuit et composition avant cuisson ((1^{ère} série d'essais) (%) | 12 | 6,3 |
| Différence MG entre produit cuit et composition avant cuisson ((2^{ème} série d'essais) (%) | 6,9 | 4,1 |

Le tableau ci-dessus permet d'avoir une idée sur la différence de matière grasse entre le produit cuit par friture et la composition avant cuisson. Cette différence permet en fait de quantifier la quantité reprise par l'étape de friture, à la fois sur le témoin mais également sur l'essai et dans les deux séries réalisées.

Au niveau du contrôle, cette différence qui peut également se qualifier de gain en matière grasse est de 12% pour la première série d'essais et de 6,9% pour la seconde.

Pour les donuts contenant de la farine de microalgues selon l'invention, ce gain de matière grasse n'est respectivement que de 6,3% pour la première série d'essais et 4,1% pour la seconde.

Ainsi comme expliqué préalablement, les donuts contenant de la farine de microalgues ont une reprise en huile lors de la phase de cuisson moins importante que les donuts fabriqués selon une composition classique, exempte de farine de microalgues.

A nouveau l'intérêt de l'invention est démontré.

### Exemple 4 : Analyse sensorielle des produits frits de type donuts obtenus selon l'exemple 3

14 personnes ont participé à l'évaluation sensorielle des produits frits de type donuts obtenus selon l'exemple précédent.

L'analyse sensorielle portait à la fois sur une évaluation visuelle des produits, mais également sur une évaluation organoleptique (texture, goût, etc..).

Concernant l'aspect visuel, la couleur du donut témoin est similaire à celle du donut contenant de la farine de microalgues selon l'invention.

La seule différence visuelle remarquée pour 9 personnes du jury est que les donuts témoins semblent avoir un aspect plus mat. Cela est lié très probablement au fait qu'ils absorbent l'huile. Les donuts contenant de la farine de microalgues selon l'invention ont été notés comme étant légèrement plus brillants en surface. Ceci pourrait s'expliquer par le fait qu'ils reprennent moins d'huile pendant la cuisson. Du coup l'huile semble former une légère pellicule extérieure leur donnant cet aspect légèrement brillant.

Cette particularité pourrait du point de vue industriel permettre peut-être de s'affranchir de l'étape de glaçage et donc encore contribuer à diminuer les coûts de production tout en diminuant également la charge calorique (pas de glaçage signifiant pas de sucre à la surface et donc une charge calorique diminuée dans le donut contenant de la farine de microalgues et n'ayant pas subi d'étape finale de glaçage au sucre).

Toujours d'un point de vue visuel, l'aspect des mies est identique sur les deux produits. La mie est d'aspect régulier et semble très souple et bien serrée.

D'un point de vue organoleptique, les deux types de donuts ont été dégustés à l'aveugle par le panel. Leur goût a été jugé très satisfaisant et agréable. Aucune différence en termes de texture, de moelleux, de goût n'a été mise en évidence. Les deux donuts ont été jugés comme ayant une texture souple, moelleuse et fondante.

Les donuts contenant de la farine de microalgues ont cependant été jugés comme étant moins gras en bouche. Ce qui est en corrélation avec le but recherché.

Ainsi, il est parfaitement possible de substituer les matières grasses d'origine végétales et/ou animales traditionnellement utilisées dans la confection de produits frits de type donuts par une farine de microalgues afin de diminuer significativement la teneur en matières grasses dans les compositions ainsi que dans les produits finis frits, sans en impacter la texture souple, moelleuse et fondante, tout en améliorant à la fois le profil nutritionnel mais également l'aspect visuel.

L'intérêt de la présente invention est ainsi démontré.

### Exemple 5 : Autres compositions pour produits frits de type donuts allégés en matières grasses selon l'invention

D'autres compositions pour produit frit selon l'invention contenant de la farine de microalgues sont été réalisées.

C'est le lot broyé à 85% de l'exemple 1 qui a été utilisé dans lesdites recettes.

La substitution de la matière grasse est telle que décrite ci-dessus dans la description : 100 parties de matière grasse habituellement utilisée d'origine végétale et/ou animale est remplacée par un mélange de 25 parties de farine de microalgues et 75 parties d'eau.

Les recettes sont données dans le tableau 2 ci-dessous.

Il n'y a pas eu de modifications dans la recette au niveau du procédé de préparation.

**Compositions : Tableau 6**

| | **TEMOIN** | | **ESSAI 1 (saccharose)** | | **ESSAI 2 (Allulose)** | |
|---|---|---|---|---|---|---|
| | **g** | **%** | **g** | **%** | **g** | **%** |
| Farine de blé | 1000,0 | 50 | 1000,0 | 50 | 970,0 | 48,5 |
| Saccharose (sucrose) | 125,0 | 6,2 | 125 | 6,2 | - | - |
| Allulose | - | - | - | - | 155 | 7,7 |
| Farine de microalgues | - | - | 25 | 1,2 | 25 | 1,2 |
| Poudre d'oeufs entiers | 50,0 | 2,5 | 50,0 | 2,5 | 50,0 | 2,5 |
| Poudre de lait écrémé | 30,0 | 1,5 | 30,0 | 1,5 | 30,0 | 1,5 |
| Levure chimique (poudre à lever) *Volcano* | 7,5 | 0,4 | 7,5 | 0,4 | 7,5 | 0,4 |
| Acide ascorbique | 0,2 | 0,0 | 0,2 | 0,0 | 0,2 | 0,0 |
| Sel | 10,0 | 0,5 | 10 | 0,5 | 10,0 | 0,5 |
| Améliorants de panification | 7,7 | 0,4 | 7,7 | 0,4 | 7,7 | 0,4 |
| Arôme vanille | 20,0 | 1 | 20,0 | 1 | 20,0 | 1 |
| Levure fraîche pressée | 70,0 | 3,5 | 70,0 | 3,5 | 70,0 | 3,5 |
| Matières grasses BISCUITINE 500 | 100 | 5 | - | - | - | - |
| Eau à 32°C+ | 580,0 | 29 | 655,00 | 32,7 | 655,0 | 32,7 |
| | **2000,4** | **100** | **2000,4** | **100** | **2000,4** | **100%** |

Le protocole de préparation est identique à celui indiqué dans l'exemple 3 précédent.

Les trois donuts obtenus selon les recettes ci-dessus, la recette témoin et celles mettant en oeuvre la farine de microalgues en remplacement de la totalité des matières grasses de type « shortening » selon la présente invention avec du saccharose ou de l'allulose en tant que sucre ont été goûtés par un panel de dégustateurs, et leur goût a été jugé très satisfaisant et agréable. Leur texture a été notée comme étant souple, moelleuse et fondante.

Il est donc tout à fait envisageable de supprimer définitivement les matières grasses d'origine végétale et/ou animale de la formule de produits frits sans en impacter ni la fabrication ni les qualités organoleptiques finales.

L'intérêt de la présente invention est ainsi démontré par les nombreux exemples ci-dessus.

## Revendications

1. Produit frit obtenu à partir d'une composition pour produit frit comprenant de la farine, de la levure, de la farine de microalgue et un liquide potable.

2. Produit frit selon la revendication 1, **caractérisé en ce que** la composition pour produit frit comprend également une matière sucrante, et de préférence du saccharose, de l'allulose et/ou leur mélanges quelconques, préférentiellement jusqu'à 20%, de préférence moins de 10%, ces pourcentages étant exprimés par rapport à la masse totale des ingrédients mis en oeuvre dans la composition.

3. Produit frit selon l'une des revendications précédentes, **caractérisée en ce que** la composition pour produit frit comprend jusqu'à 80%, de préférence entre 10% et 80%, préférentiellement 20% et 70%, plus préférentiellement entre 30% et 65%, et encore plus préférentiellement entre 35% et 55% de farine, ces pourcentages étant exprimés par rapport à la masse totale des ingrédients mis en oeuvre dans la composition, et/ou jusqu'à 30%, de préférence moins de 20%, plus préférentiellement moins de 10%, et encore plus préférentiellement moins de 5% d'oeufs ou ovoproduits, ces pourcentages étant exprimés par rapport à la masse totale des ingrédients mis en oeuvre dans la composition, et/ou jusqu'à 30%, de préférence moins de 20%, plus préférentiellement moins de 10%, et encore plus préférentiellement moins de 5% de dérivés du lait, ces pourcentages étant exprimés par rapport à la masse totale des ingrédients mis en oeuvre dans la composition.

4. Produit frit selon l'une quelconque des revendications précédentes, **caractérisé en ce qu'**il ne contient pas de gluten.

5. Produit frit selon l'une quelconque des revendications précédentes, **caractérisé en ce que** les matières grasses d'origine végétale et/ou animale d'origine ont été remplacées partiellement ou totalement par de la farine de microalgues, préférentiellement totalement.

6. Produit frit selon l'une quelconque des revendications précédentes, **caractérisé en ce que** la teneur en matière grasse de la composition pour produit frit est réduite de 30%, de préférence de 40% et encore plus préférentiellement de 55% en comparaison de la teneur en matière grasse contenue dans la composition pour produit frit exempte de farine de microalgues, ces pourcentages étant exprimés par rapport à la masse totale des ingrédients mis en oeuvre dans la composition.

7. Produit frit selon l'une quelconque des revendications précédentes, **caractérisé en ce qu'**une substitution de 100 parties de matière grasse habituellement utilisée d'origine végétale et/ou animale dans la composition pour produit frit est remplacée par un mélange de 25 parties de farine de microalgues et 75 parties d'un liquide potable, ledit liquide potable étant avantageusement de l'eau, ladite eau pouvant être de l'eau de source, de l'eau minérale, gazéifiée naturellement ou par adjonction de gaz carbonique ou non gazéifiée.

8. Produit frit selon l'une quelconque des revendications précédentes, caractérisé en que la composition pour produit frit comprend de 0,5% à 20% de farine de microalgues, de préférence de 1% à 10%, plus préférentiellement de 1% à 5%, ces pourcentages étant exprimés par rapport à la masse totale des ingrédients mis en oeuvre dans la composition.

9. Produit frit selon l'une quelconque des revendications précédentes, **caractérisé en ce que** la somme des composants consistant en la farine de microalgues et le liquide potable dans la composition pour produit frit représente 25%, et de préférence au moins 30% en masse de la totalité des ingrédients utilisés dans ladite composition.

10. Produit frit selon l'une quelconque des revendications précédentes, dans lequel la farine de microalgues est une farine de microalgues du genre *Chlorella,* et plus particulièrement de l'espèce *Chlorella protothecoides.*

11. Produit frit selon l'une quelconque des revendications précédentes, dans lequel la farine de microalgues contient au moins 12%, au moins 25%, au moins 40%, au moins 50%, ou au moins 75% en poids sec de lipides, et/ou dont la teneur en matière grasse est réduite d'au moins 30%, de préférence, 40% et encore plus préférentiellement de 45% par rapport au produit frit obtenu selon une composition exempte de farine de microalgues.

12. Produit frit selon la revendication précédente, **caractérisé en ce qu'**il est sucré et qu'il contient de préférence du saccharose, de l'allulose et/ou leurs mélanges quelconques, et/ou **caractérisé en ce qu'**il est saupoudré de sucre, glacé, glacés au sucre fondu, recouvert d'un nappage sucré et aromatisé, et/ou fourré d'une crème pâtissière, de chantilly salée ou sucrée et/ou aromatisée, d'une ganache, d'un fourrage gras.

13. Utilisation d'une farine de microalgues comme matière grasse de substitution (« fat replacer ») dans la préparation d'un produit frit selon l'une quelconque des revendications 1 à 12.

14. Procédé de préparation d'un produit frit selon l'une quelconque des revendications 1 à 12, selon lequel de la farine, des oeufs ou ovoproduits, de la levure et de la farine de microalgues sont mélangés à un liquide potable, avant cuisson, ledit procédé comprenant avantageusement les étapes suivantes:
- une étape de mélange de la farine, des oeufs ou ovoproduits, de la levure et de la farine de microalgues à un liquide potable, préférentiellement de l'eau ;
- une étape de fermentation de ce mélange,
- une étape de mise en forme dudit mélange en boules de pâte,
- une étape de cuisson desdites boules de pâtes dans un bain d'huile chaude entre 150°C et 200°C pendant une durée allant de 30 secondes à 3 minutes pour chaque face.

15. Procédé de préparation selon la revendication précédente permettant d'obtenir des produits frits possédant une teneur en matières grasses totales réduite d'au moins 30%, de préférence, 40% et encore plus préférentiellement de 45% par rapport au produit frit obtenu selon une composition exempte de farine de microalgues.

## Patentansprüche

1. Frittiertes Produkt, erhalten von einer Zusammensetzung für ein frittiertes Produkt, umfassend Mehl, Hefe, Mikroalgenmehl und eine trinkbare Flüssigkeit.

2. Frittiertes Produkt gemäß Anspruch 1, **dadurch gekennzeichnet, dass** die Zusammensetzung für ein frittiertes Produkt ebenfalls einen Süßstoff und vorzugsweise Saccharose, Allulose und/oder beliebige Mischungen davon, vorzugsweise bis zu 20%, bevorzugt weniger als 10% umfasst, wobei diese Prozentangaben bezogen auf das Gesamtgewicht der in der Zusammensetzung verwendeten Bestandteile ausgedrückt sind.

3. Frittiertes Produkt gemäß einem der vorhergehenden Ansprüche, **dadurch gekennzeichnet, dass** die Zusammensetzung für ein frittiertes Produkt bis zu 80%, vorzugsweise zwischen 10% und 80%, bevorzugt zwischen 20% und 70%, bevorzugter zwischen 30% und 65% und noch bevorzugter zwischen 35% und 55% Mehl, wobei diese Prozentangaben bezogen auf das Gesamtgewicht der in der Zusammensetzung verwendeten Bestandteile ausgedrückt sind, und/oder bis zu 30%, vorzugsweise weniger als 20%, bevorzugter weniger als 10% und noch bevorzugter weniger als 5% Eier oder Eiprodukte, wobei diese Prozentangaben bezogen auf das Gesamtgewicht der in der Zusammensetzung verwendeten Bestandteile ausgedrückt sind, und/oder bis zu 30%, vorzugsweise weniger als 20%, bevorzugter weniger als 10% und noch bevorzugter weniger als 5% Milchderivate umfasst, wobei diese Prozentangaben bezogen auf das Gesamtgewicht der in der Zusammensetzung verwendeten Bestandteile ausgedrückt sind.

4. Frittiertes Produkt gemäß einem der vorhergehenden Ansprüche, **dadurch gekennzeichnet, dass** es kein Gluten enthält.

5. Frittiertes Produkt gemäß einem der vorhergehenden Ansprüche, **dadurch gekennzeichnet, dass** die Fette pflanzlichen Ursprungs und/oder tierischen Ursprungs durch Mikroalgenmehl teilweise oder vollständig, bevorzugt vollständig, ersetzt worden sind.

6. Frittiertes Produkt gemäß einem der vorhergehenden Ansprüche, **dadurch gekennzeichnet, dass** der Fettgehalt der Zusammensetzung für ein frittiertes Produkte um 30%, bevorzugt um 40% und noch bevorzugter um 55% verglichen mit dem Fettgehalt reduziert ist, der in der Zusammensetzung für ein frittiertes Produkt frei von Mikroalgenmehl enthalten ist, wobei diese Prozentangaben bezogen auf das Gesamtgewicht der in der Zusammensetzung verwendeten Bestandteile ausgedrückt sind.

7. Frittiertes Produkt gemäß einem der vorhergehenden Ansprüche, **dadurch gekennzeichnet, dass** eine Substitution 100 Teile des üblicherweise verwendeten Fetts pflanzlichen und/oder tierischen Ursprungs in der Zusammensetzung für ein frittiertes Produkt durch eine Mischung von 25 Teilen Mikroalgenmehl und 75 Teilen einer trinkbaren Flüssigkeit ersetzt, wobei besagte trinkbare Flüssigkeit vorteilhafterweise Wasser ist, wobei besagtes Wasser Quellwasser, Mineralwasser mit natürlichem Kohlensäuregehalt oder mit Kohlensäure versetztes Wasser oder stilles Wasser sein kann.

8. Frittiertes Produkt gemäß einem der vorhergehenden Ansprüche, **dadurch gekennzeichnet, dass** die Zusammensetzung für ein frittiertes Produkt 0,5% bis 20% Mikroalgenmehl, bevorzugt 1% bis 10%, bevorzugter 1% bis 5% umfasst, wobei diese Prozentangaben bezogen auf das Gesamtgewicht der in der Zusammensetzung verwendeten Bestandteile ausgedrückt sind.

9. Frittiertes Produkt gemäß einem der vorhergehenden Ansprüche, **dadurch gekennzeichnet, dass** die Summe der Bestandteile, bestehend aus Mikroalgenmehl und trinkbarer Flüssigkeit, in der Zusammensetzung für ein frittiertes Produkt 25 Gew.-% und bevorzugt wenigstens 30 Gew.-% der gesamten in besagter Zusammensetzung verwendeten Bestandteile darstellt.

10. Frittiertes Produkt gemäß einem der vorhergehenden Ansprüche, wobei das Mikroalgenmehl ein Mehl von Mikroalgen der Gattung *Chlorella* und insbesondere der Spezies *Chlorella protothecoides* ist.

11. Frittiertes Produkt gemäß einem der vorhergehenden Ansprüche, wobei das Mikroalgenmehl wenigstens 12 Trockengew.-%, wenigstens 25 Trockengew.-%, wenigstens 40 Trockengew.-%, wenigstens 50 Trockengew.-% oder wenigstens 75 Trockengew.-% Lipide enthält und/oder der Fettgehalt davon wenigstens um 30%, bevorzugt um 40% und noch bevorzugter um 45% bezogen auf das frittierte Produkt reduziert ist, das gemäß einer Zusammensetzung frei von Mikroalgenmehl erhalten wird.

12. Frittiertes Produkt gemäß dem vorhergehenden Anspruch, **dadurch gekennzeichnet, dass** es gesüßt ist und dass es vorzugsweise Saccharose, Allulose und/oder beliebige Mischungen davon enthält, und/oder **dadurch gekennzeichnet, dass** es mit Zucker bestreut, glasiert, mit geschmolzenem Zucker glasiert, mit einem gesüßten und aromatisiertem Guss überzogen ist und/oder mit einer Patisserie-Creme, mit gesalzener oder gesüßter und/oder aromatisierter Schlagsahne, mit einer Ganache, mit einer fetthaltigen Füllung gefüllt ist.

13. Verwendung eines Mikroalgenmehls als Fettersatz ("fat replacer") in der Herstellung eines frittierten Produkts gemäß einem der Ansprüche 1 bis 12.

14. Verfahren zur Herstellung eines frittierten Produkts gemäß einem der Ansprüche 1 bis 12, wobei das Mehl, die Eier oder Eiprodukte, die Hefe und das Mikroalgenmehl mit einer trinkbaren Flüssigkeit vor dem Backen gemischt werden, wobei besagtes Verfahren vorteilhafterweise die folgenden Schritte umfasst:
- einen Schritt des Mischens des Mehls, der Eier oder Eiprodukte, der Hefe und des Mikroalgenmehls mit einer trinkbaren Flüssigkeit, vorzugsweise Wasser;
- einen Schritt der Gärung dieser Mischung,
- einen Schritt des Formens besagter Mischung zu Teigkugeln,
- einen Schritt des Backens besagter Teigkugeln in einem heißen Ölbad zwischen 150°C und 200°C über einen Zeitraum von 30 Sekunden bis 3 Minuten für jede Seite.

15. Verfahren zur Herstellung gemäß dem vorhergehenden Anspruch, das den Erhalt der frittierten Produkte mit einem Gesamtfettgehalt erlaubt, der wenigstens um 30%, bevorzugt um 40% und noch bevorzugter um 45% bezogen auf das frittierte Produkt reduziert ist, das gemäß einer Zusammensetzung frei von Mikroalgenmehl erhalten wird.

## Claims

1. A fried product obtained from a composition for a fried product comprising flour, yeast, microalgal flour and a drinkable liquid.

2. The fried product according to claim 1, **characterized in that** the composition for a fried product also comprises a sweetening substance, and preferably saccharose, allulose and/or any mixtures thereof, preferentially up to 20%, preferably less than 10%, these percentages being expressed relative to the total weight of the ingredients used in the composition.

3. The fried product according to one of the preceding claims, **characterized in that** the composition for a fried product comprises up to 80%, preferably between 10% and 80%, preferentially between 20% and 70%, more preferentially between 30% and 65%, and even more preferentially between 35% and 55%, of flour, these percentages being expressed relative to the total weight of the ingredients used in the composition, and/or up to 30%, preferably less than 20%, more preferentially less than 10%, and even more preferentially less than 5%, of eggs or egg products, these percentages being expressed relative to the total weight of the ingredients used in the composition, and/or up to 30%, preferably less than 20%, more preferentially less than 10%, and even more preferentially less than 5%, of milk derivatives, these percentages being expressed relative to the total weight of the ingredients used in the composition.

4. The fried product according to any one of the preceding claims, **characterized in that** it does not contain gluten.

5. The fried product according to any one of the preceding claims, **characterized in that** the fats of vegetable origin and/or animal origin have been partially or totally replaced by the microalgal flour, preferentially totally.

6. The fried product according to any one of the preceding claims, **characterized in that** the fat content of the composition for a fried product is reduced by 30%, preferably by 40%, and even more preferentially by 55%, in comparison with the fat content contained in the composition for a fried product free of microalgal flour, these percentages being expressed relative to the total weight of the ingredients used in the composition.

7. The fried product according to any one of the preceding claims, **characterized in that** 100 parts of fat normally used, of vegetable and/or animal origin, in the composition for a fried product are replaced by a mixture of 25 parts of microalgal flour and 75 parts of a drinkable liquid, said drinkable liquid being advantageously water, said water possibly being spring water, mineral water, water that is naturally sparkling or sparkling through the addition of carbon dioxide, or still.

8. The fried product according to any one of the preceding claims, **characterized in that** the composition for a fried product comprises from 0.5% to 20% of microalgal flour, preferably from 1% to 10%, more preferentially from 1% to 5%, these percentages being expressed relative to the total weight of the ingredients used in the composition.

9. The fried product according to any one of the preceding claims, **characterized in that** the sum of the components consisting of the microalgal flour and the drinkable liquid in the composition for a fried product represents 25%, and preferably at least 30%, by weight of all of the ingredients used in said composition.

10. The fried product according to any one of the preceding claims, wherein the microalgal flour is a flour of microalgae of the genus *Chlorella,* and more particularly of the species *Chlorella protothecoides.*

11. The fried product according to any one of the preceding claims, wherein the microalgal flour contains at least 12%, at least 25%, at least 40%, at least 50%, or at least 75% by dry weight of lipids, and/or the fat content of which is reduced by at least 30%, preferably by 40%, and even more preferentially by 45%, relative to the fried product obtained according to a composition free of microalgal flour.

12. The fried product according to the preceding claim, **characterized in that** it is sweet and that it preferably contains saccharose, allulose and/or any mixtures thereof, and/or **characterized in that** it is dusted with sugar, glazed, iced with melted sugar, covered with a sweet and flavored topping, and/or filled with a confectioner's custard, with savory or sweet and/or flavored Chantilly cream, with a ganache or with a fatty filling.

13. The use of a microalgal flour as fat replacer in the preparation of a fried product according to any one of claims 1 to 12.

14. A process for preparing a fried product according to any one of claims 1 to 12, wherein the flour, the eggs or egg products, the yeast and the microalgal flour are mixed with a drinkable liquid, before cooking, said process advantageously comprising the following steps:
- a step of mixing the flour, the eggs or egg products, the yeast and the microalgal flour with a drinkable liquid, preferentially water,
- a step of fermenting this mixture,
- a step of shaping said mixture into dough balls,
- a step of cooking said dough balls in a bath of hot oil between 150°C and 200°C for a period ranging from 30 seconds to 3 minutes for each side.

15. The preparation process according to the preceding claim making it possible to obtain fried products having a total fat content reduced by at least 30%, preferably by 40%, and even more preferentially by 45%, relative to the fried product obtained according to a composition free of microalgal flour.
